(19) Europäisches Patentamt
European Patent Office
Office européen des brevets

(11) EP 1 760 103 B1

(12) **EUROPEAN PATENT SPECIFICATION**

(45) Date of publication and mention of the grant of the patent:
**28.11.2012 Bulletin 2012/48**

(51) Int Cl.:
***C08G 61/12*** *(2006.01)* ***C07D 409/12*** *(2006.01)*
***C07D 333/20*** *(2006.01)* ***C07D 307/52*** *(2006.01)*

(21) Application number: **06018137.7**

(22) Date of filing: **30.08.2006**

(54) **Arylamine polymer and organic thin film transistor**

Arylaminpolymer und organischer Dünnschichttransistor

Polymère arylamine et transistor à couche mince organique

(84) Designated Contracting States:
**DE FR**

(30) Priority: **30.08.2005 JP 2005249285**
**09.03.2006 JP 2006064169**

(43) Date of publication of application:
**07.03.2007 Bulletin 2007/10**

(73) Proprietor: **Ricoh Company, Ltd.**
**Tokyo 143-8555 (JP)**

(72) Inventors:
- **Okada, Takashi**
**Tokyo 143-8555 (JP)**
- **Sasaki, Masaomi**
**Tokyo 143-8555 (JP)**
- **Torii, Masafumi**
**Tokyo 143-8555 (JP)**
- **Kawamura, Shinichi**
**Tokyo 143-8555 (JP)**
- **Sagisaka, Toshiya**
**Tokyo 143-8555 (JP)**
- **Akiyama, Yoshikazu**
**Tokyo 143-8555 (JP)**
- **Yamaga, Takumi**
**Tokyo 143-8555 (JP)**

(74) Representative: **Barz, Peter**
**Meissner Bolte**
**Widenmayerstrasse 48**
**80538 München (DE)**

(56) References cited:
**WO-A-00/79617** **WO-A-03/035714**

- **YAMAMOTO, KIMIHISA ET AL: "Synthesis and Electrochemical Properties of Oligo- and Poly (thienylphenylamine)s" MACROMOLECULES , 35(15), 5782-5788 CODEN: MAMOBX; ISSN: 0024-9297, 2002, XP002410561**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 760 103 B1

## Description

The present invention relates to an arylamine polymer, and an organic transistor having an organic semiconductor layer containing the arylamine polymer.

## Discussion of the Background

Various kinds of functional elements such as photoelectric conversion elements, thin layer transistor elements and luminous elements are proposed based on luminous characteristics and carrier transport characteristics of organic materials. Elements using such organic materials are expected to fully utilize merits thereof, for example, light weigh, flexibility, inexpensiveness and low manufacturing cost.

Among these functional elements, various kinds of low molecular weight materials and polymers are proposed for photoelectric conversion elements, especially as materials for solar batteries and carrier transport materials for use in electrophotography. Further efficiency is demanded for the low molecular weight material and high speed performance and durability are demanded for the polymers.

In addition, various kinds of low molecular weight materials and polymers are proposed as materials for luminous elements. For example, the efficiency of low molecular weight materials is improved by adopting a wide variety of layered structures. The durability is improved by desirably controlling doping method.

However, in the case of such a low molecular weight material, i.e., a collective of low molecular weight materials, it is reported that the status of the layer changes when the low molecular weight material is used for an extended period of time. That is, such a low molecular weight material has an essential problem of the stability of a layer over time.

On the other hand, with regard to the polymer materials, intensive studies have been made on poly-p-phenylenevinylene (PPV) series, poly-thiophen, etc.

However, high performance luminous elements have not been obtained because of unsolved drawbacks such as difficulty in improving purity of the material and low fluorescence quantum yield.

In spite of these drawbacks, further improvements have been made on the polymer materials. This is because, since polymer materials are stable in a glass state, an excellent luminous element can be manufactured when the fluorescence quantum yield is improved.

Polymer materials having an arylamine unit as a repeating unit are specified as such polymer materials, for example in WO99/20675, WO97/09394, WO03/035714, published unexamined Japanese patent applications Nos. H10-310635 and H08-157575 and Synth. Met., 84, 269 (1997).

In addition, with regard to organic transistor elements, various kinds of low molecular weight materials and polymer materials are proposed. For example, pentacene, phthalocyanine, fullerene, anthradithiophene, thiophene oligomer, bisdithienothiophene are specified as the low molecular weight materials, and polythiophene, polythienylenevinylene and polyarylamine are specified as the polymer materials.

The polymer materials specified in the documents are greatly improved for the mobility, which is an important characteristic for materials for organic electronics. However, polymer materials having a higher mobility are demanded in terms of application for organic electronics, especially organic field electric transistors (FET).

Further, to apply organic materials having characteristics of low cost manufacturing, sufficient flexibility and strength, light weight and possible increase in size to various kinds of functional elements (photoelectric conversion elements, transistor elements and luminous elements) and fully utilize the characteristics thereof, it is desired that the organic materials are sufficiently soluble in an organic solvent. However, pi-conjugated polymers having a characteristic structure of elongated conjugations are inflexible in general. This inflexible structure is a cause of lowering the solubility of such pi-conjugated polymers.

The polymermaterials specified in the documents mentioned above tend to have a difficulty in solubility thereof. Wide-ranging molecular designs have been attempted to solve this drawback relating to the solubility.

The present inventors already found that polymer materials having an arylamine unit (including ethylene) as a repeating unit having a pi-conjugated linkage as its main chain have an excellent luminous characteristic and are useful for an organic electroluminescence (EL) having an excellent durability and an active layer of an organic transistor. However, in consideration of application of such a polymer material to organic electronic elements, especially organic field-effect transistor (FET) elements, the mobility is desired to be further improved.

In addition, the study and development of organic thin film (TFT) transistors using organic semiconductor materials has been active in recent years. Thin layers of organic semiconductor materials can be easily formed by an easy method such as a printing method and a spin coating method using a wet process. Thin layers of organic semiconductor materials also have an advantage over a thin layer formed of inorganic semiconductor materials in that the temperature of the manufacturing process can be lowered. Thereby, it is possible to form a thin layer on a plastic substrate generally having a low thermal durability so that electronic devices such as display can be reduced in weight and cost. Further, the latitude of designing is broad because of the flexibility of such a plastic substrate.

JOP H05-55568 describes an acene based materials, e.g., pentacene, as an organic semiconductor material. The organic transistorusingpentacene as an organic semiconductor layer is reported to have a relatively high mobility. However, these acene based materials have an extremely low solubility in a typical solvent. Therefore, when such an acene based material is used to form a thin organic semiconductor layer of an organic transistor, a vacuum deposition method is desired to be used. Therefore, one of the merits of organic semiconductor materials, i.e., easy manufacturing methods such as coating and printing, is not fully utilized.

In addition, as organic polymer semiconductor materials, Appl. Phys. Lett., 69(26), 4108 (1996) and Science, 290, 2123 (2000) describe poly(3-alkylthiophen) and a copolymer of dialkylfulolene and bithiophene, respectively. Since these organic semiconductor materials have a low but sufficient solubility by introducing of alkyl groups, a thin layer thereof can be formed not by a vacuum deposition process but by coating and printing. These polymeric organic semiconductor materials have a high mobility in a state in which molecules are arranged in order. However, the arrangement state of the molecules depends on the kinds of solvents and coating methods. As a result, this leads to a problem of variation of the characteristics of transistors and deficiency of reproducibility of the characteristics.

Yamamoto, K. et al., "Synthesis and Electrochemical Properties of Oligo- and Poly(thienylphenylamine)s", Macromolecules, 35(15), 5782-5788, describes the preparation of oligo- and poly(thienylphenylamine)s by the oxidative polymerization of tris(thienylphenyl)amine derivatives. In a scheme trimer and tetramer products are shown.

WO 00/79617 A relates to a method for forming an electronic device having a semiconducting active layer comprising a polymer, the method comprising aligning the chains of the polymer parallel to each other by bringing the polymer into a liquid-crystalline phase.

## SUMMARY OF THE INVENTION

Because of these reasons, the present inventors recognize that a need exists for an arylamine polymer having an excellent mobility characteristic and which can be inexpensively manufactured and used as a material for use in organic electronic materials such as luminous elements, FET elements, and photoelectric conversion elements, and for an organic thin film transistor which can be manufactured by an easymethod such as coating and printing and has a good reproducibility. The mobility characteristic represented in the present invention is a mobility characteristic of a charge based on the positive hole transfer.

Accordingly, an object of the present invention is to provide an arylamine polymer having an excellent mobility characteristic and which can be inexpensively manufactured and used as a material for use in organic electronic materials and an organic thin film transistor which can be manufactured by an easy method such as coating and printing and has a good reproducibility.

Briefly these objects and other objects of the present invention as hereinafter described will become more readily apparent and can be attained, either individually or in combination thereof, by an arylamine polymer containing a repeating unit represented by the following chemical structure (I),

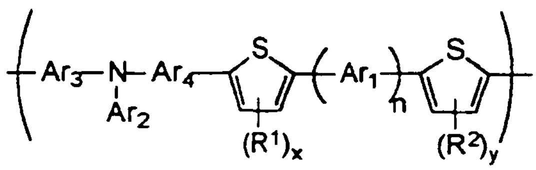

··· (I)

In the chemical structure (I), $Ar_1$, $Ar_3$, and $Ar_4$ independently represent a substituted or non-substituted divalent aromatic hydrocarbon group, $Ar_2$ represents a substituted univalent aromatic hydrocarbon group, $R^1$ and $R^2$ independently represent a hydrogen atom, a substituted or non-substituted alkyl group, a substituted or non-substituted alkoxy group or a substituted or non-substituted alkylthio group, x and y independently represent an integer of from 0 to 2, and n represents 1.

It is preferred that, in the arylamine mentioned above, the repeating unit is represented by the following chemical structure (II),

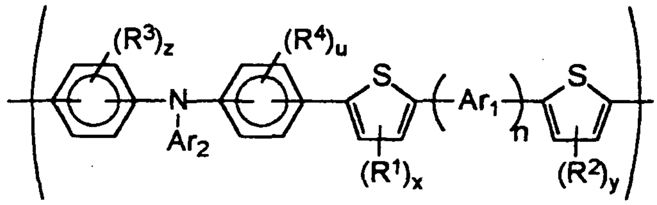

...(II)

wherein $Ar_1$ represents a substituted or non-substituted divalent aromatic hydrocarbon group, $Ar_2$ represents a substituted univalent aromatic hydrocarbon group, $R^1$, $R^2$, $R^3$ and $R^4$ independently represent a hydrogen atom, a substituted or non-substituted alkyl group, a substituted or non-substituted alkoxy group, or a substituted or non-substituted alkylthio group, x and y independently represent an integer of from 0 to 2, z and u independently represent an integer of from 0 to 4, and n represents 1.

It is still further preferred that, in the arylamine mentioned above, the repeating unit is represented by the following chemical structure (III),

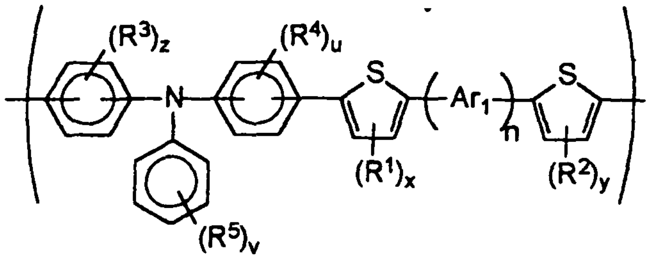

...(III)

wherein $Ar_1$ independently represents a substituted or non-substituted divalent aromatic hydrocarbon group, $R^1$, $R^2$, $R^3$, and $R^4$, independently represent a hydrogen atom, a substituted or non-substituted alkyl group, a substituted or non-substituted alkoxy group, or a substituted or non-substituted alkylthio group, $R^5$ represents a substituted or non-substituted alkyl group, a substituted or non-substituted alkoxy group, or a substituted or non-substituted alkylthio group, x and y independently represent an integer of from 0 to 2, z and u independently represent an integer of from 0 to 4, v represents an integer of from 1 to 5 and n represents 1.

As another aspect of the present invention, an organic thin film transistor is provided which contains an organic semiconductor layer containing the arylamine mentioned above.

It is preferred that the organic thin film transistor further includes a structure including a pair of electrodes for applying a current through the organic semiconductor layer and a third electrode.

It is still further preferred that, in the organic thin film transistor, the third electrode and the structure are provided with an insulating layer therebetween.

These and other objects, features and advantages of the present invention will become apparent upon consideration of the following description of the preferred embodiments of the present invention taken in conjunction with the accompanying drawings.

## BRIEF DESCRIPTION OF THE DRAWINGS

Various other objects, features and attendant advantages of the present invention will be more fully appreciated as the same becomes better understood from the detailed description when considered in connection with the accompanying drawings in which like reference characters designate like corresponding parts throughout and wherein:

Figs. 1A to 1D are schematic diagrams illustrating the organic thin film transistor related to the present invention;
Fig. 2 is a diagram illustrating an infrared absorption spectrum of an example polymer of the present invention;
Fig. 3 is a diagram illustrating an infrared absorption spectrum of another example polymer of the present invention;
Fig. 4 is a diagram illustrating an infrared absorption spectrum of an example polymer of the present invention;
Fig. 5 is a diagram illustrating an infrared absorption spectrum of a reference example polymer;
Fig. 6 is a diagram illustrating an infrared absorption spectrum of an example polymer of the present invention;
Fig. 7 is a diagram illustrating an infrared absorption spectrum of another reference example polymer;
Fig. 8 is a diagram illustrating an infrared absorption spectrum of an example polymer of the present invention;

Fig. 9 is a diagram illustrating an infrared absorption spectrum of another example polymer of the present invention;
Fig. 10 is a diagram illustrating the measuring results of the transistor characteristics of a manufactured example of the present invention;
Fig. 11 is a diagram illustrating the measuring results of the transistor characteristics of another manufactured example of the present invention;
Fig. 12 is a diagram illustrating the measuring results of the transistor characteristics of another manufactured example of the present invention;
Fig. 13 is a diagram illustrating the measuring results of the transistor characteristics of another manufactured example of the present invention;
Fig. 14 is a diagram illustrating the measuring results of the transistor characteristics of a manufactured reference example ;
Fig. 15 is a diagram illustrating the measuring results of the transistor characteristics of another manufactured example of the present invention; and
Fig. 16 is a diagram illustrating the measuring results of the transistor characteristics of another manufactured example of the present invention.

## DETAILED DESCRIPTION OF THE INVENTION

The present invention is described in detail below with reference to drawings.

The arylamine polymers of the present invention are polymeric materials including the repeating unit represented by the following chemical structures (I) to (III):

[Chemical Structure I]

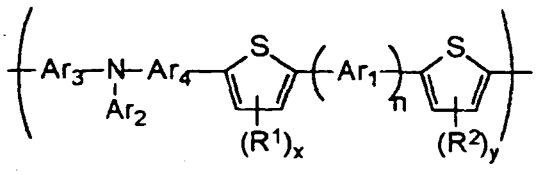

···(I)

[Chemical Structure II]

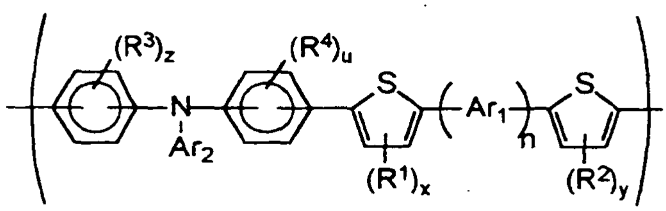

···(II)

[Chemical Structure III]

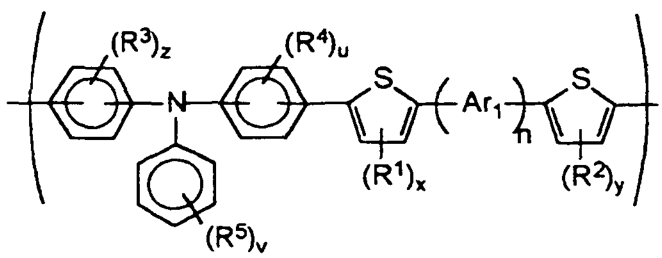

···(III)

The arylamine polymers of the present invention can be used as charge transfer polymers for organic transistors,

polymers for use in organic thin film electroluminescence elements or materials for use in organic electronics for electrophotographic image bearing member materials.

Below are descriptions of the method of manufacturing the arylamines of the present invention.

For example, a cross coupling reaction (Suzuki Coupling) in which a halogenated aryl compound and a boron aryl compound are used and another cross coupling reaction (Stille Coupling) in which a halogen aryl compound and a tin aryl compound are used can be used. Known manufacturing methods can be used to manufacture the arylamine polymer.

A method of manufacturing the arylamine polymer of the present invention using Suzuki Coupling is described. The arylamine polymer of the present invention is generally obtained by heating a solution in which a boronic acid compound or an ester of a boronic acid and a halogen compound are stoichiometrically equally present under the presence of a palladium catalyst and a base to proceed a polymerization reaction. In addition, a random copolymer can be obtained by adding multiple kinds of boronic acid compounds or halogen compounds in the reaction process. Thereby, it is possible to adjust characteristics of the arylamine polymer.

Halogen atoms contained in the halogenated aryl compound are preferably iodine atom or bromine atom in terms of reactivity.

An aryl boronic acid or an ester thereof is used as the boron aryl compound. An ester of an aryl boronic acid is preferred since the ester of an aryl boronic acid does not produce a cyclic anhydride (boroxine) formed of a trimer, which is produced when an aryl boronic acid is used. In addition, when an ester of an aryl boronic acid is used, the synthesized compound has a high crystalline property and purification thereof is easy.

Specific examples of synthesizing an ester of aryl boronic acid are as follows:

(i) Heat an aryl boronic acid and an alkane diol in an organic anhydrate solvent;
(ii) Metalize a halogen portion of a halogenated aryl compound and add an ester of an alkoxyboron thereto;
(iii) Prepare a Grignard reagent of an aryl halogen and add an ester of an alkoxyboron thereto; and
(iv) Heat a halogenated aryl compound and bis(pinacolate) diboron or bis(neopentyl glycolate)diboron under the presence of a palladium catalyst.

Specific examples of the palladium catalysts include $Pd(PPh_3)_4$, $PdCl_2(PPh_3)_2$, $Pd(OAC)_2$, $PdCl_2$ and a catalyst in which triphenyl phosphine is added to palladium-carbon as a ligand. $Pd(PPh_3)_4$ is most commonly used.

Abase is required for this reaction. Good results are obtained when a relatively weak base such as $Na_2CO_3$, $NaHCO_3$ and $K_2CO_3$ is used. A strong base such as $Ba(OH)_2$ and $K_3PO_4$ is effective for addressing problems such as steric hindrance. Other specific examples thereof include caustic soda, caustic potash and metal alcoxides such as potassium t-butoxide, sodium t-butoxide, lithium t-butoxide, potassium 2-methyl-2-butoxide, sodium 2-methyl-2-butoxide, sodium methoxide, sodium ethoxide, potassium ethoxide and potassium methoxide.

In addition, to smoothly proceed the reaction, it is possible to use a phase transfer catalyst. For example, tetraalkyl halogenated ammonium, tetraalkyl ammonium hydrogen sulfate and tetraalkyl ammonium hydroxide can be used. Preferred specific examples thereof include tetra-n-butylhalogenated ammonium, benzyl triethyl halogenated ammonium and tricaprylylmethyl ammonium chlorinate.

Specific examples of the reaction solvents include alcohols and ethers such as methanol, ethanol, isopropanol, butanol, 2-methoxyethanol, 1,2-dimethoxyethane, bis(2-methoxyethyl) ether; cyclic ethers such as dioxane and tetrahydrofuran; benzene, toluene, xylene, dimethylsulfoxide, N,N-dimethylformamide, N-methylpyrrolidone, and 1,3-dimethyl-2-imidazolidinone.

The reaction temperature of the polymerization mentioned above is determined depending on the reactivity of a monomer or a reaction solvent used but is preferred to be set at a temperature not higher than the solvent.

The reaction time in the polymerization reaction mentioned above can be determined depending on the reactivity of a monomer used or the molecular weight of a desired polymer and preferably ranges from 2 to 50 hours and more preferably from 5 to 24 hours.

It is also possible to add a molecular weight controlling agent to control the molecular weight in the polymerization process or an endcapping agent as an endmodifying group for a polymer to have its end. These agents can be added in the reaction process or at when the reaction starts. Therefore, a group based on an endcapping agent can be bonded at the end of the polymer of the present invention.

A compound having one pharmacophore such as phenyl boronic acid, bromobenzene and benzene iodide can be used as the molecular weight controlling agent or the endcapping agent.

The polymer of the present invention preferably has an average molecular weight of from 1, 000 to 1, 000, 000 in polystyrene conversion number and more preferably, from 2,000 to 500,000. Themolecularweight that is too small may cause practical problems such as cracking, i.e., the deterioration the layer forming property. The molecular weight that is too large may also cause practical problems such as deterioration of solubility in a typical organic solvent, resulting in the increase in the viscosity of the solvent, which leads to the difficulty in application of the solvent.

In addition, it is also possible to add a small amount of a branching agent during polymerization to improve the mechanical characteristics of the arylamine polymer. Compounds having three same or different pharmacophores can be used as the branching agent and these can be used alone or in combination.

The thus obtained arylamine polymer of the present invention is used after removing materials used for polymerization, such as the base, non-reactive monomers and the endcapping agents, and impurities, such as inorganic salts generated during the polymerization. In this purification process, known methods such as reprecipitation, extraction, soxhlet extraction, extracorporeal ultrafiltration (ECUM) and dialysis can be used.

Specific examples of the thus obtained polymers having the repeating units represented by the chemical structures (I) to (III) are as follows.

A univalent substituted aromatic hydrocarbon group $Ar_2$ in the chemical structure (I) can be a monocyclic or a polycyclic group (condensation polycyclic group, non-condensation polycyclic group). Specific examples there of include phenyl group, naphthyl group, vinylene group, fluorenyl group, azulenyl group, anthryl group, triphenylenyl group, glycenyl group, biphenyl group and terphenyl group. Divalent groups of the substituted aromatic hydrocarbon groups mentioned above can be used as the substituted or non-substituted divalent aromatic hydrocarbon groups $Ar_1$, $Ar_3$ and $Ar_4$ in the chemical structure (I).

These groups ($Ar_1$, $Ar_2$, $Ar_3$ and $Ar_4$) having a cyclic structure can have various kinds of substituent groups as follows:

(1) halogen atoms, trifluoromethyl group, cyano group, nitro group;
(2) straight chained or branch chained alkyl groups or alkoxy groups having 1 to 25 carbon atoms; these can be further substituted by halogen atom, cyano group, phenyl group, hydroxyl group, carboxyl group, alkoxy group and alkylthio group;
(3) aryloxy groups (having phenyl group and naphthyl group as aryl group. These can contain a halogen atom as a substituent group and also straight chained or branch chained alkyl groups, alkoxy groups and alkylthio groups having 1 to 25 carbon atoms. Specific examples thereof include phenoxy group, 1-naphthyloxy group, 2-naphthyloxy group, 4-methylphenoxy group, 4-methoxyphenoxy group, 4-chlorophenoxy group, and 6-methyl-2-naphthyloxy group);
(4) alkylthio groups or arylthio groups (Specific examples thereof include methylthio group, ethylthio group, phenylthio group, p-methylphenylthio group.);
(5) alkyl substituted amino groups (Specific examples thereof include diethyl amino group, N-methyl-N-phenyl amino group, N, N-diphenyl amino group, N,N-di(p-tolyl) amino group, dibenzyl amino group, piperidino group, morpholino group, and julolidyl group.); and acyl groups (Specific examples thereof include acetyl group, propionyl group, butyryl group, malonyl group and benzoyl group).

The arylamine polymers having the repeating units represented by the chemical structures (I) to (III) can have a halogen atom, a substituted or non-substituted alkyl group, or a substituted or non-substituted alkoxy group on their aromatic rings as substituent groups. In terms of improvement of the solubility of the arylamine polymers in a solvent, it is preferred to have a substituted or non-substituted alkyl group, a substituted or non-substituted alkoxy group or a substituted or non-substituted alkylthio group.

When the number of carbon atoms of these substituent groups increases, the solubilityof the arylamine polymers increases. However, the charge transport property thereof deteriorates. Therefore, it is preferred to select substitutional groups which can impart desired characteristics to the arylamine polymer in a range in which the solubility does not deteriorate. Preferred specific examples of such suitable substituent groups include alkyl group, alkoxy group or alkylthio group having 1 to 25 carbon atoms. The same or different substitutional groups can be independently introduced. In addition, these alkyl groups, alkoxy groups and alkylthio groups can further contain halogen atoms, cyano groups, aryl groups, hydroxyl groups, carboxyl groups, and aryl groups substituted by straight chained, branch chained or cyclic alkyl groups, alkoxy groups or alkylthio groups having 1 to 12 carbon atoms.

Specific examples of the alkyl groups include methyl group, ethyl group, n-propyl group, i-propyl group, t-butyl group, s-butyl group, n-butyl group, i-butyl group, pentyl group, hexyl group, heptyl group, octyl group, nonyl group, decyl group, 3, 7-dimethyloctyl group, 2-ethylhexyl group, trifluoromethyl group, 2-cyanoethyl group, benzyl group, 4-chlorobenzyl group, 4-methyl benzyl group, cyclopentyl group and cyclohexyl group. Specific examples of the alkoxy groups and the alkylthio groups include groups in which an oxygen atom or a sulfur atom is introduced into the linking position of the alkyl groups mentioned above, respectively.

Next, the organic thin film transistor of the present invention is described.

Transistor structure

Figs. 1A to 1D are diagrams illustrating schematic structures of the organic thin filmtransistor of the present

invention. An organic semiconductor layer 1 of the organic thin film transistor of the present invention contains the arylamine polymer having one of the repeating units illustrated in the chemical structures (I) to (III). The organic transistor of the present invention includes a source electrode 2, a drain electrode 3 and a gate electrode 4, all of which are spatially separated from each other. As illustrated in Figs. 1C and 1D, an insulating layer 5 can be provided between the gate electrode 4 and the organic semiconductor layer 1. In the organic thin film transistor, the current flowing in the organic semiconductor layer 1 between the source electrode 2 and the drain electrode 3 is controlled by the voltage applied to the gate electrode 4.

The organic thin film transistor of the present invention can be provided on a substrate. For example, commonly-used substrates such as glass, silicon and plastic can be used. In addition, when an electroconductive substrate is used, the electroconductive substrate itself can function as a gate electrode. Further, a gate electrode can be accumulated on a substrate. Among these substrates, a plastic sheet is preferred in consideration of characteristics, for example, flexibility, reduction in weight, inexpensive cost and anti-shocking, desired for a device to which the organic transistor of the present invention is applied.

Specific examples of such plastic sheets include films formed of polyethylene terephthalate, polyethylene naphthalate, polyether sulfone, polyether imides, polyether etherketones polyphenyl sulfides, polyarylates, polyimides, polycarbonates, cellulose triacetate, cellulose acetate propionate, etc.

Coating Method: Organic Semiconductor Layer 1

A solution in which the arylamine polymers mentioned above of the present invention is dissolved in a solvent, for example dichloromethane, tetrahydrofuran, chloroform, toluene, dichlorobenzene and xylene, can be coated on the substrate to form a thin layer, i.e., the organic semiconductor layer 1.

Specific examples of the methods of coating the organic semiconductor layer 1 include a spray coating method, a spin coating method, a blade coating method, a dip coating method, a casting method, a roll coating method, a bar coating method, a dye coating method, an ink j et method and a dispense method. A good combination is suitably selected among the methods mentioned above of forming a layer and the solvents mentioned above depending on the materials.

The thin film formed by these coating methods has excellent strength, toughness and durability and is free from cracking. Such thin film is suitable for organic electronics such as photoelectric conversion elements, Field effect transistor (FET) elements and luminescent elements.

In the organic thin film transistor of the present invention, there is no specific limit to the layer thickness of the organic semiconductor layer 1. The layer thickness is set to be in a range in which a uniform layer (i.e., there is no gap and/or hole having an adverse effect on the carrier transport property of the organic semiconductor layer 1) canbe formed. The layer thickness of the organic semiconductor layer 1 is typically not greater than 1 μm and preferably from 5 to 200 nm.

Insulating Layer

The insulating layer 5 for use in the organic transistor of the present invention can be formed of various kinds of insulating materials. Specific examples thereof include inorganic insulating materials such as silica, silicon nitride, aluminum oxide, aluminum nitride, titan oxide, tantalum oxide, tin oxide, vanadium oxide, barium strontium titanate, zirconium oxidized barium titanate, zirconic acid lead titanate, lead lanthanum titanate, strontium titanate, barium titanate, barium magnesium fluoride, bismuth niobium acid tantalate, and trioxide yttrium. In addition, polymers such as polyimide, polyvinylalcohol, polyvinylphenol, polyester, polyethylene, polyphenylene sulfide, non-substituted or halogen atom substituted polyparaxylylene, polyacrylonitrile and cyanoethyl pullulan can be also used. These can be used alone or in combination. There is no specific limit to the selection of the insulating materials but it is preferred to select an insulating material having a high dielectric constant and a low electroconductivity.

Specific examples of the methods of manufacturing the insulating layer using the materials mentioned above include dry deposition processes such as a chemical vacuum deposition (CVD) method, a plasma CVDmethod and a plasma polymerizationmethod, and wet coating processes such as a spray coating method, a spin coating method, a dip coating method, an inkjet method, a casting method, a blade coating method and a bar coating method.

Interface between Organic Semiconductor and Insulating Layer, e.g., hexamethyldisilazane (HMDS)

In the organic thin film transistor of the present invention, an organic thin layer can be provided between the insulating layer and the organic semiconductor layer to improve the adhesiveness thereof, decrease the gate voltage and reduce the leak current. There is no specific limit to the organic thin layer as long as the organic thin layer does not have a chemical effect on the organic semiconductor layer. For example, an organic molecular film and a polymer thin

layer can be used.

As the organic molecular film, coupling agents such as octadecyl trichlorosilane and hexamethylene disilazane can be used. In addition, in the case of the polymer thin layer, the polymer insulating materials mentioned above can be used. These can function as a kind of an insulating layer. This organic thin layer can be subject to an anisotropic treatment by rubbing.

Electrode

There is no specific limit to the gate electrode, the source electrode and the drain electrode for use in the organic thin film transistor of the present invention as long as these electrodes are electroconductive materials. Specific examples thereof include platinum, gold, silver, nickel, chromium, copper, iron, tin, antimony, lead, tantalum, indium, aluminum, zinc, magnesium, alloys thereof, electroconductive metal oxides such as indium/tin oxides, organic and inorganic semiconductors in which electroconductivity is improved by doping, etc., such as silicon single crystal, polysilicon, amorphous silicon, germanium, graphite, polyacetylene, polyparaphenylene, polythiophene, polypyrrol, polyaniline, polythienylene vinylene, polyparaphenylene vinylene, and complex of polyethylene dioxythiophene and polystyrene sulfonic acid.

Among the electroconductive materials mentioned above, materials having a low electroconductivity at the contact phase with the semiconductor layer are preferred for the source electrode.

Specific examples of forming an electrode include: a method in which a known method such as a photolithographic method and liftoff technology is used to form an electrode from an electroconductive thin layer formed of the material mentioned above by a method such as deposition and sputtering; and a method in which an electrode is formed by etching a resist on a metal foil of, for example, aluminum and copper, by thermal transfer, ink jet, etc. In addition, it is possible to form an electrode by patterning a solution or liquid dispersion of an electroconductive polymer or a liquid dispersion of electroconductive particulates by direct ink jet. Lithography and laser application can be used to form an electrode from a coated layer. It is also possible to use a method in which ink, electroconductive paste, etc. containing electroconductive polymers and/or electroconductive particulates are patterned by a printing method such as intaglio plating, anastatic printing, planographic printing and screen printing.

Extraction Electrode and Protective Layer

The organic thin film transistor of the present invention can have an extraction electrode from each electrode if desired.

The organic thin film transistor of the present invention can be stably operated in the atmosphere. If desired, a protective layer can be provided in terms of protection from mechanical destruction and moisture and/or gas and convenience for integration of a device. Applied Device

The organic thin film transistor of the present invention can be utilized as elements for driving image display elements such as liquid crystal, organic electroluminescence and electrophoretic migration. When such elements are integrated, it is possible to manufacture a display referred to as "electronic paper". It is also possible to use ICs in which the organic thin film transistors are integrated as a device such as IC tags.

Having generally described preferred embodiments of this invention, further understanding can be obtained by reference to certain specific examples which are provided herein for the purpose of illustration only and are not intended to be limiting. In the descriptions in the following examples, the numbers represent weight ratios in parts, unless otherwise specified.

## EXAMPLES

The present invention is described with reference to Examples but not limited thereto.

Below are synthetic examples of polymers for use in the present invention.

Example 1

Synthesis of Polymer 1

Polymer 1 is prepared by polymerization under the following conditions along with the following reaction process 1.

Reaction process 1

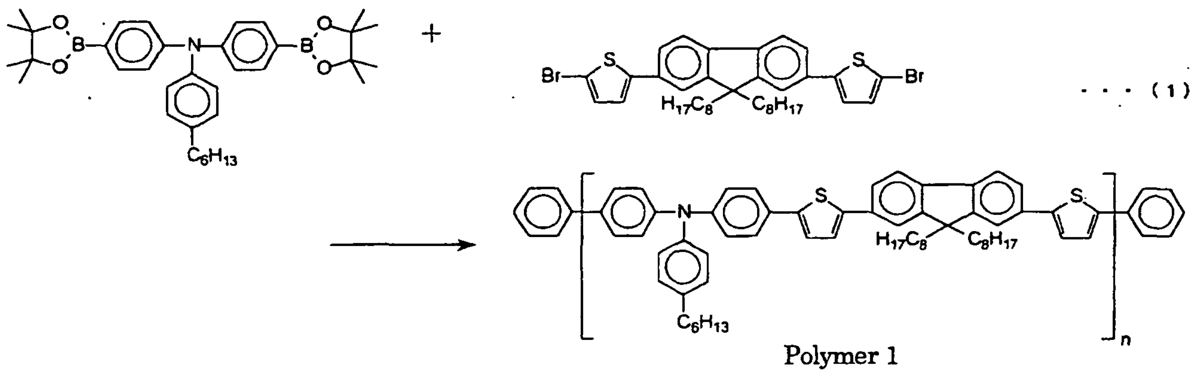

· · · (1)

Polymer 1

Place the following components in a 100 ml flask.

| | |
|---|---|
| Diboron ester derivative illustrated in the reaction process 1 | 0.872 g (1.5 mmol) |
| Dibromo derivative illustrated in the reaction process 1 | 1.069 g (1.5 mmol) |
| Aliquat 336 (manufactured by Sigma-Aldrich Corp.) as a phase transfer catalyst | 12.1 mg (0.03 mmol) |
| Phenyl boronic acid | 5.5 mg (0.045 mmol) |

Add 4.33 mg (0.00375 mmol) of tetrakis triphenyl phosphine palladium and 11 ml of toluene thereto. Subsequent to nitrogen gas replacement, add 3.1 ml of 2M-sodium carbonate. After a 17 hour reflux, add 118 mg (0.75mmol) of bromobenzene for termination reaction followed by an 8 hour flux. After cooling down the reaction solution to room temperature, drop the organic layer therein in a mixture solvent of methanol and water for re-precipitation to obtain a polymer. Prepare a dichloromethane solution of the polymer and sufficiently wash the solution with deionized water. Prepare a tetrahydrofuran solution thereof and drop the solution in methanol for re-precipitation to purify the polymer. The yield of the thus obtained polymer 1 is 1.20 g and the yield ratio thereof is 91 %.

The number average molecular weight of the polymer 1 in polystyrene conversion number measured by Gel Permeation Chromatography (GPC) is 48,200. The weight average molecular weight thereof is 134,600.

The glass transition temperature of the polymer 1 obtained using differential scanning calorimetry (DSC) is 122.1 °C.

Elemental analysis (calculated values in parenthesis): C: 83.04 % (83.23 %), H: 8.05 % (7.90%), N: 1.53 % (1.59 %), S: 7.49 % (7.28 %).

Infrared absorption spectrum (NaCl casting film) of the polymer 1 is illustrated in Fig. 2.

Example 2

Synthesis of Polymer 2

Polymer 2 is prepared by polymerization under the following conditions along with the following reaction process 2.

Reaction process 2

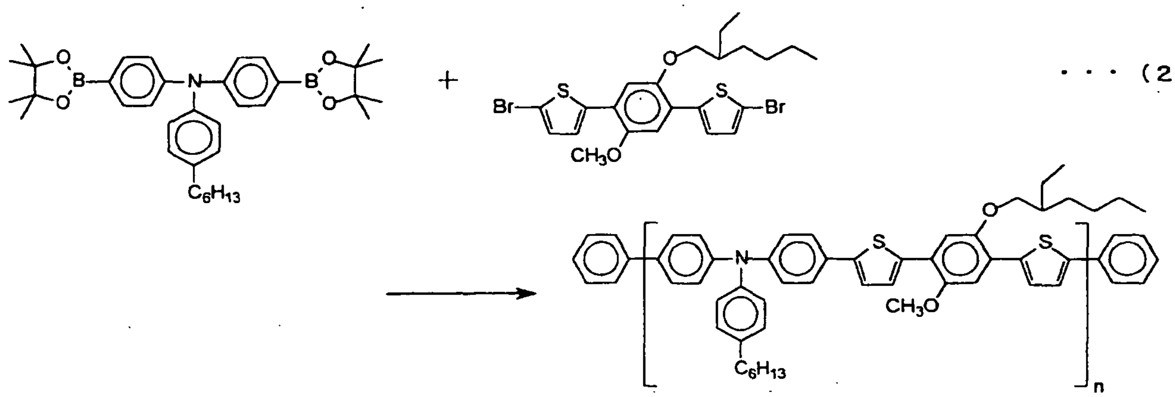

· · · (2)

Polymer 2

Place the following components in a 100 ml flask.

| | |
|---|---|
| Diboron ester derivative illustrated in the reaction process 2 | 0.872 g (1.5 mmol) |
| Dibromo derivative illustrated in the reaction process 2 | 0.838 g (1.5 mmol) |
| Aliquat 336 (manufactured by Sigma-Aldrich Corp.) as a phase transfer catalyst | 12.1 mg (0.03 mmol) |
| Phenyl boronic acid | 5.5 mg (0.045 mmol) |

Add 4.33 mg (0.00375 mmol) of tetrakis triphenyl phosphine palladium and 11 ml of toluene thereto. Subsequent to nitrogen gas replacement, add 3.1 ml of 2M-sodium carbonate. After a 20 hour reflux, add 118 mg (0.75 mmol) of bromobenzene for termination reaction followed by a 6 hour flux. After cooling down the reaction solution to room temperature, drop the organic layer therein in a mixture solvent of methanol and water for re-precipitation to obtain a polymer. Prepare a dichloromethane solution of the polymer and sufficiently wash the solution with de-ionized water. Prepare a tetrahydrofuran solution thereof and drop the solution in methanol for re-precipitation to purify the polymer. The yield of the thus obtained polymer 2 is 1.07 g and the yield ratio thereof is 98 %.

The number average molecular weight of the polymer 2 in polystyrene conversion number measured by Gel Permeation Chromatography (GPC) is 5,900. The weight average molecular weight thereof is 11,800.

The glass transition temperature of the polymer 2 obtained using differential scanning calorimetry (DSC) is 108.3 °C.

Elemental analysis (calculated values in parenthesis): C: 77.46 % (77.75 %), H: 7.12 % (7.08 %), N: 1.79 % (1.93 %), S: 8.69 % (8.83 %).

Infrared absorption spectrum (NaCl casting film) of the polymer 2 is illustrated in Fig. 3.

Example 3

Synthesis of Polymer 3

Polymer 3 is prepared by polymerization under the following conditions along with the following reaction process 3.

Reaction process 3

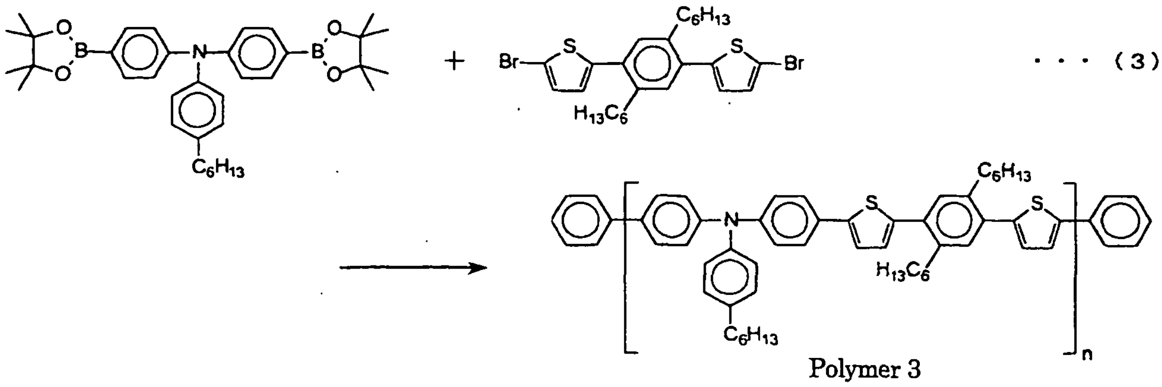

· · · (3)

Polymer 3

Place the following components in a 100 ml flask.

| | |
|---|---|
| Diboron ester derivative illustrated in the reaction process 3 | 0.872 g (1.5 mmol) |
| Dibromo derivative illustrated in the reaction process 3 | 0.853 g (1.5 mmol) |
| Aliquat 336 (manufactured by Sigma-Aldrich Corp.) as a phase transfer catalyst | 12.1 mg (0.03 mmol) |
| Phenyl boronic acid | 5.5 mg (0.045 mmol) |

Add 4.33 mg (0.00375 mmol) of tetrakis triphenyl phosphine palladium and 11 ml of toluene thereto. Subsequent to nitrogen gas replacement, add 3.1 ml of 2M-sodium carbonate. After a 30 hour reflux, add 118 mg (0.75 mmol) of bromobenzene for termination reaction followed by a 6 hour flux. After cooling down the reaction solution to room temperature, drop the organic layer therein in a mixture solvent of methanol and water for re-precipitation to obtain a polymer. Prepare a dichloromethane solution of the polymer and sufficiently wash the solution with de-ionized water. Prepare a tetrahydrofuran solution thereof and drop the solution in methanol for re-precipitation to purify the polymer. The yield of the thus obtained polymer 3 is 1.10 g and the yield ratio thereof is 99 %.

The number average molecular weight of the polymer 3 in polystyrene conversion number measured by Gel Permeation Chromatography (GPC) is 8,500. The weight average molecular weight thereof is 19,400.

The glass transition temperature of the polymer 3 obtained using differential scanning calorimetry (DSC) is 84.1 °C.

Elemental analysis (calculated values in parenthesis): C: 81.30 % (81.58 %), H: 7.94 % (7.80 %), N: 1.84 % (1.90 %), S: 8.48 % (8.71 %).

Infrared absorption spectrum (NaCl casting film) of the polymer 3 is illustrated in Fig. 4.

Reference Example 1

Synthesis of Polymer 4

Polymer 4 is prepared by polymerization under the following conditions along with the following reaction process 4.

Reaction process 4

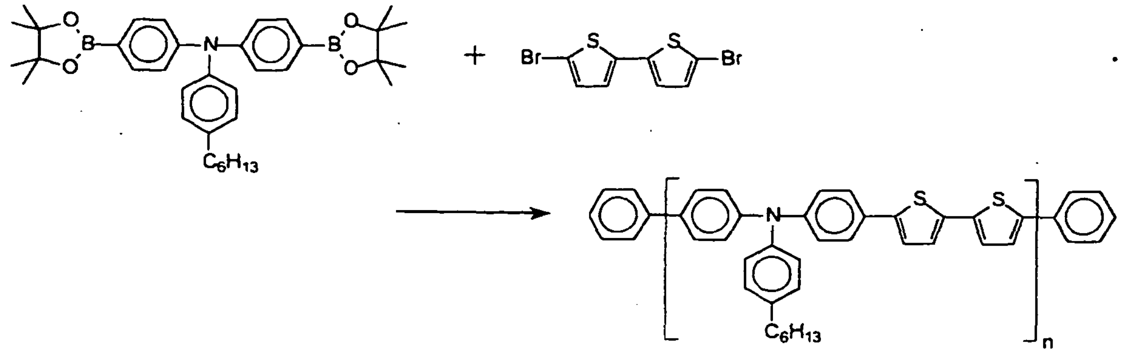

···(4)

Polymer 4

Place the following components in a 100 ml flask.

| | |
|---|---|
| Diboron ester derivative illustrated in the reaction process 4 | 1.182 g (2.03 mmol) |
| Dibromo derivative illustrated in the reaction process 4 | 0.659 g (2.03 mmol) |
| Aliquat 336 (manufactured by Sigma-Aldrich Corp.) as a phase transfer catalyst | 16.4 mg (0.03 mmol) |
| Phenyl boronic acid | 7.4 mg (0.06 mmol) |

Add 5.87 mg (0.0051 mmol) of tetrakis triphenyl phosphine palladium and 14 ml of toluene thereto. Subsequent to nitrogen gas replacement, add 4.1 ml of 2M-sodium carbonate. After a 14 hour reflux, add 157 mg (1.0 mmol) of bromobenzene for termination reaction followed by a 6 hour flux. After cooling down the reaction solution to room temperature, drop the organic layer therein in a mixture solvent of methanol and water for re-precipitation to obtain polymer 4. The obtained polymer is not soluble in tetrahydrofuran and chloroform. The yield of the polymer 4 is 1.00 g and the yield ratio thereof is 100 %.

Elemental analysis (calculated values in parenthesis): C: 77.90 % (78.17 %), H: 5.87 % (5.94 %), N: 2.73 % (2.85 %), S: 13.02 % (13.04 %).

Infrared absorption spectrum (KBr tablet method) of the polymer 4 is illustrated in Fig. 5.

Example 4

Synthesis of Polymer 3-2

Polymer 3-2 is prepared by polymerization under the following conditions along with the following reaction process 3-2.

Reaction process 3-2

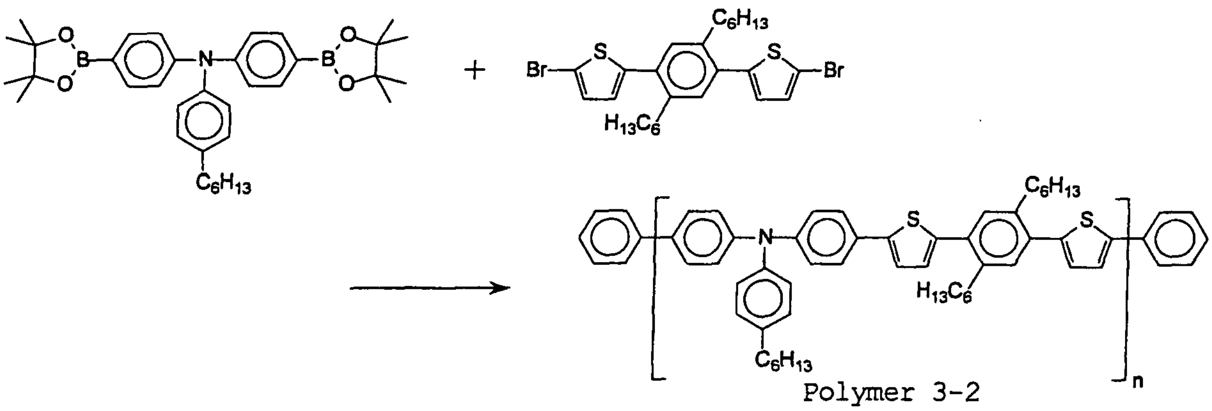

Polymer 3-2

Place the following components in a 50 ml flask.

| | |
|---|---|
| Diboron ester derivative illustrated in the reaction process 3-2 | 0.872 g (1.5 mmol) |
| Dibromo derivative illustrated in the reaction process 3-2 | 0.853 g (1.5 mmol) |
| Aliquat 336 (manufactured by Sigma-Aldrich Corp.) as a phase transfer catalyst | 12.1 mg (0.03 mmol) |

Add 8.7 mg (0.00752 mmol) of tetrakis triphenyl phosphine palladium and 6 ml of toluene thereto. Subsequent to nitrogen gas replacement, add 3.5 ml of 2M-sodium carbonate. After a 13 hour reflux, for termination reaction, add 73 mg (0.6 mmol) of phenyl boroic acid followed by a 5 hour reflux, and further add 109 mg (0.69 mmol) of bromobenzene followed by a 4 hour flux. After cooling down the reaction solution to room temperature, drop the organic layer therein in a mixture solvent of methanol and water for re-precipitation to obtain a polymer. Prepare a chloroform solution of the polymer and sufficiently wash the solution with de-ionizedwater. Prepare a tetrahydrofuran solution thereof and drop the solution in methanol for re-precipitation to purify the polymer. The yield of the thus obtained polymer 3-2 is 1.07 g and the yield ratio thereof is 97 %.

The number average molecular weight of the polymer 3-2 in polystyrene conversion number measured by Gel Permeation Chromatography (GPC) is 42,600. The weight average molecular weight thereof is 164,600.

Elemental analysis (calculated values in parenthesis): C: 81.42 % (81.58 %), H: 8.01 % (7.80 %), N: 1.92 % (1.90 %), S: 8.64 % (8.71 %).

Infrared absorption spectrum (NaCl casting film) of the polymer 3 is illustrated in Fig. 6.

Reference Example 2

Synthesis of Polymer 5

Polymer 5 is prepared by polymerization under the following conditions along with the following reaction process 5.

Reaction process 5

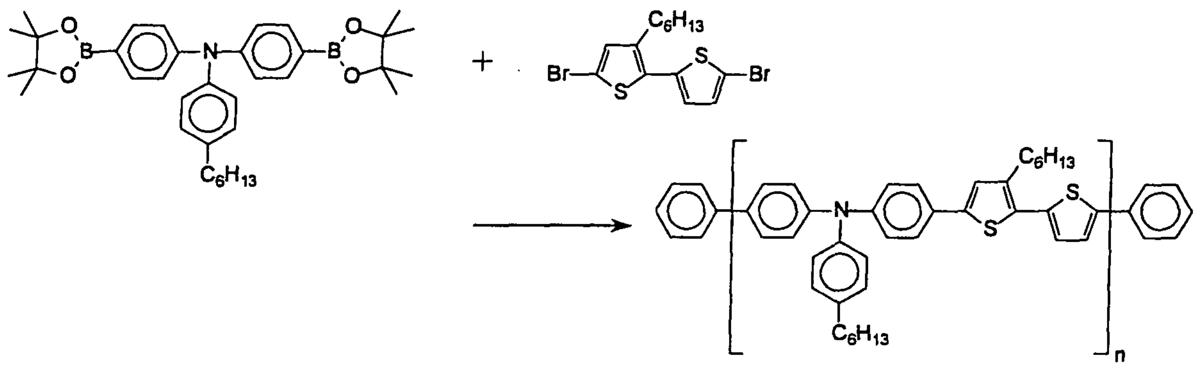

Polymer 5

Place the following components in a 50 ml flask.

| | |
|---|---|
| Diboron ester derivative illustrated in the reaction process 5 | 0.872 g (1.5 mmol) |
| Dibromo derivative illustrated in the reaction process 5 | 0.612 g (1.5 mmol) |
| Aliquat 336 (manufactured by Sigma-Aldrich Corp.) as a phase transfer catalyst | 13.4 mg (0.033 mmol) |

Add 8.7 mg (0.0075 mmol) of tetrakis triphenyl phosphine palladium and 6.2 ml of toluene thereto. Subsequent to nitrogen gas replacement, add 3.5 ml of 2M-sodium carbonate. After a 13 hour reflux, for termination reaction, add 73 mg (0.6 mmol) of phenyl boroic acid followed by a 5 hour reflux, and further add 109 mg (0.69 mmol) of bromobenzene followed by a 4 hour flux. After cooling down the reaction solution to room temperature, drop the organic layer therein in a mixture solvent of methanol and water for re-precipitation to obtain a polymer. Prepare a chloroform solution of the polymer and sufficiently wash the solution with de-ionized water. Prepare a tetrahydrofuran solution thereof and drop the solution inmethanol for re-precipitation to purify the polymer. The yield of the thus obtained polymer 5 is 0.844 g and the yield ratio thereof is 98 %.

The number average molecular weight of the polymer 5 in polystyrene conversion number measured by Gel Permeation Chromatography (GPC) is 40,900. The weight average molecular weight thereof is 176,400.

Elemental analysis (calculated values in parenthesis): C: 78.98 % (79.26 %), H: 7.22 % (7.18 %), N: 2.57 % (2.43 %), S: 11.04 % (11.13 %).

Infrared absorption spectrum (NaCl casting film) of the polymer 5 is illustrated in Fig. 7.

Example 5

Synthesis of Polymer 6

Polymer 6 is prepared by polymerization under the following conditions along with the following reaction process 6.

Reaction process 6

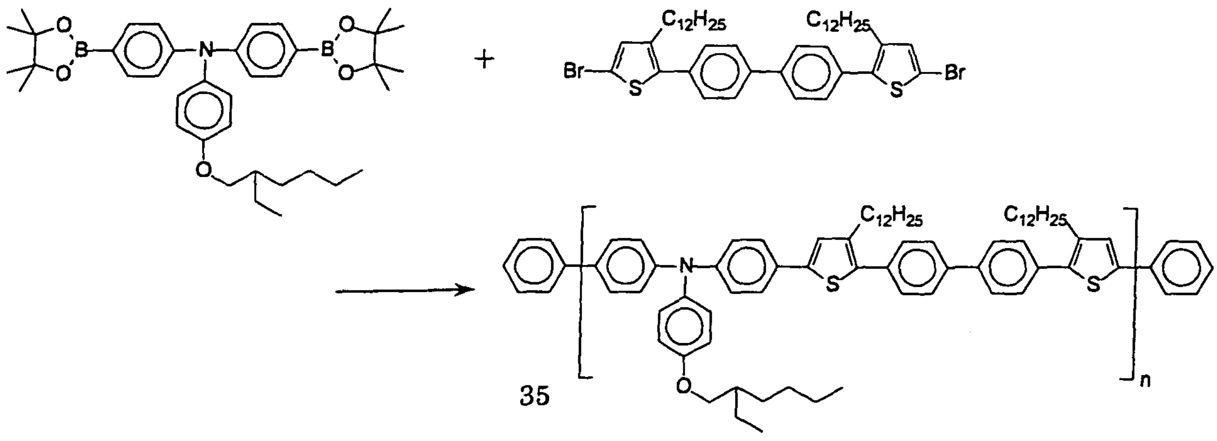


Polymer 6

21-05-2

Place the following components in a 50 ml flask.

| | |
|---|---|
| Diboron ester derivative illustrated in the reaction process 6 | 0.938 g (1.5 mmol) |
| Dibromo derivative illustrated in the reaction process 6 | 1.219 g (1.5 mmol) |
| Aliquat 336 (manufactured by Sigma-Aldrich Corp.) as a phase transfer catalyst | 12.6 mg (0.03 mmol) |

Add 8.7 mg (0.00752 mmol) of tetrakis triphenyl phosphine palladium and 6.2 ml of toluene thereto. Subsequent to nitrogen gas replacement, add 3. 5 ml of 2M-sodium carbonate. After a 4 hour reflux, for termination reaction, add 73 mg (0.6 mmol) of phenyl boroic acid followed by a 3 hour reflux, and further add 118 mg (0.75 mmol) of bromobenzene followed by a 3 hour flux. After cooling down the reaction solution to room temperature, drop the organic layer therein in a mixture solvent of methanol and water for re-precipitation to obtain a polymer. Prepare a chloroform solution of the polymer and sufficiently wash the solution with de-ionized water. Prepare a tetrahydrofuran solution thereof and drop the solution in methanol for re-precipitation to purify the polymer. The yield of the thus obtained polymer 6 is 1.23 g and the yield ratio thereof is 80 %.

The number average molecular weight of the polymer 6 in polystyrene conversion number measured by Gel Permeation Chromatography (GPC) is 35, 700. The weight average molecular weight thereof is 184,300.

Elemental analysis (calculated values in parenthesis): C: 82.35 % (82.60 %), H: 8.61 % (8.76 %), N: 1.45 % (1.37 %), S: 6.10 % (6.26 %).

Infrared absorption spectrum (NaCl casting film) of the polymer 6 is illustrated in Fig. 8.

Example 6

Synthesis of Polymer 7

Polymer 7 is prepared by polymerization under the following conditions along with the following reaction process 7.

Reaction process 7

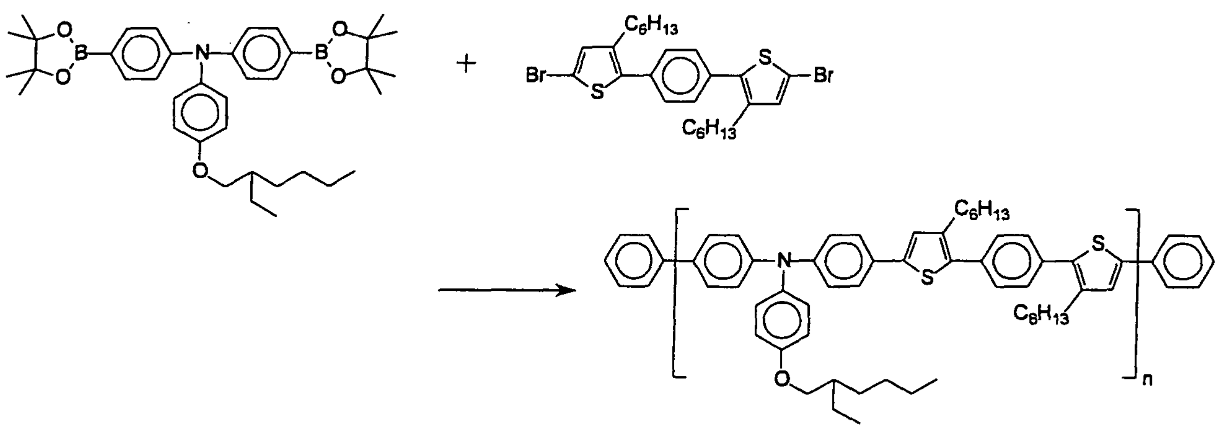

Polymer 7

Place the following components in a 50 ml flask.

| | |
|---|---|
| Diboron ester derivative illustrated in the reaction process 7 | 0.938 g (1.5 mmol) |
| Dibromo derivative illustrated in the reaction process 7 | 0.852 g (1.5 mmol) |
| Aliquat 336 (manufactured by Sigma-Aldrich Corp.) as a phase transfer catalyst | 12.8 mg (0.03 mmol) |

Add 8.7 mg (0.00752 mmol) of tetrakis triphenyl phosphine palladium and 6.2 ml of toluene thereto. Subsequent to nitrogen gas replacement, add 3. 5 ml of 2M-sodium carbonate. After a 4 hour reflux, for termination reaction, add 73 mg (0.6 mmol) of phenyl boroic acid followed by a 3 hour reflux, and further add 118 mg (0.75 mmol) of bromobenzene followed by a 3 hour flux. After cooling down the reaction solution to room temperature, drop the organic layer therein in a mixture solvent of methanol and water for re-precipitation to obtain a polymer. Prepare a chloroform solution of the polymer and sufficiently wash the solution with de-ionized water. Prepare a tetrahydrofuran solution thereof and drop the solution in methanol for re-precipitation to purify the polymer. The yield of the thus obtained polymer 7 is 0.76 g and the yield ratio thereof is 65 %.

The number average molecular weight of the polymer 7 in polystyrene conversion number measured by Gel Permeation Chromatography (GPC) is 29,700. The weight average molecular weight thereof is 154,700.

Elemental analysis (calculated values in parenthesis): C: 79.87 % (80.05 %), H: 7.99 % (7.88 %), N: 1.74 % (1.80 %), S: 8.35 % (8.22 %).

Infrared absorption spectrum (NaCl casting film) of the polymer 7 is illustrated in Fig. 9.

Example of Manufacturing Substrate for Evaluating Organic Thin Film Transistor

Form an insulating layer of $SiO_2$ having a thickness of 200 nm by thermal oxidizing the surface of a silicon substrate having a 30 mmp-doped square. Cover one side thereof with a resist film (TSMR8800 manufactured by Tokyo Ohka Kogyo Co., Ltd.) and remove the oxidized film on the other side by fluorinated acid. Deposit aluminum having a thickness of 300 nm on the side fromwhich the oxidized film is removed. Thereafter, remove the resist film by acetone to obtain a substrate for evaluating an organic thin film transistor.

Example 7

The following thin film transistor is manufactured on the substrate for evaluating an organic thin film transistor manufactured by the method described above using the polymer 1.

Spin-coat and dry a solution formed of a mixture solvent of tetrahydrofuran (THF) and paraxylene with a ratio of 8 to 2 containing the polymer 1 in an amount of about 1 % by weight based on the weight of the solution on the substrate to manufacture an organic semiconductor layer having a layer thickness of 30 nm.

Deposit gold to form a source electrode and a drain electrode having a layer thickness of 100 nm so that an organic thin film transistor can have a channel length of 30 $\mu$m and a channel width of 10 mm.

Further, to confirm the reproducibility of the characteristics of the organic thin filmtransistor, another organic thin filmtransistor is manufactured in the same manner.

The thus manufactured organic thin film transistors have a structure illustrated in Fig. 1D. The p-doped silicon substrate is used as a gate electrode together with an aluminum thin layer provided to the bottom of the substrate.

The electric field-effect mobility, which is the characteristic of the organic thin film transistor, is measured.

The measuring results of the transistor characteristic of this device are illustrated in Fig. 10. In Fig. 10, $V_{ds}$ represents the source-drain voltage.

The following relationship is used to calculate the electric field-effect mobility of the organic thin film transistor.

$$I_{ds} = \mu C_{in} W (V_G - V_{TH})^2 / 2L$$

In the relationship, $C_{in}$ represents the capacitance per unit area of the gate insulating layer, W represents the channel width, L represents the channel length, $V_G$ represents the gate voltage, $I_{ds}$ represents the source-drain current ($I_d$ in Figs. 9 to 16 is the same as $I_{DS}$), and $\mu$ represents the mobility, $V_{TH}$ represents a threshold voltage of the gate above which the channel starts being formed.

The electric field-effect mobility of the manufactured organic thin film transistors is $2.4 \times 10^{-3}$ cm$^2$/Vs and $1.9 \times 10^{-3}$ cm$^2$/Vs.

There are few variances among the elements with regard to the transistors. It can be concluded that the reproducibility of the transistor characteristic of these transistors is good.

Example 8

Two organic thin film transistors are manufactured in the same manner as in Example 7 except that spin coating is performed using a toluene solution containing the polymer 1 in an amount of 1.0 % by weight based on the weight of the solution.

The electric field-effect mobility of the manufactured organic transistors measured is $1.9 \times 10^{-3}$ cm$^2$/Vs and $2.0 \times 10^{-3}$ cm$^2$/Vs.

There are few variances among the elements with regard to the transistors and the reproducibility of the transistor characteristic of these transistors is good.

As seen in Examples 7 and 8, it is found that an organic thin film transistor having a good reproducibility and few characteristic variances can be provided regardless of different solvents.

Example 9

Two organic thin film transistors are manufactured in the same manner as in Example 7 except that spin coating is performed using a solution of a mixture solvent of tetrahydrofuran (THF) and paraxylene with a ratio of 8 to 2 containing the polymer 2 in an amount of 1.0 % by weight based on the weight of the solution.

The measuring results of the transistor characteristics of the device are illustrated in Fig. 11.

The electric field-effect mobility of the manufactured organic transistors measured is $4.2 \times 10^{-4}$ cm$^2$/Vs and $4.3 \times 10^{-4}$ cm$^2$/Vs.

There are few variances among the elements of the transistors and these transistors have transistor characteristics with a good reproducibility.

Example 10

Two organic thin film transistors are manufactured in the same manner as in Example 7 except that spin coating is performed using a toluene solution containing the polymer 2 in an amount of 1.0 % by weight based on the weight of the solution.

The electric field-effect mobility of the manufactured organic transistors measured is $4.3 \times 10^{-4}$ cm$^2$/Vs and $4.1 \times 10^{-4}$ cm$^2$/Vs.

There are few variances among the elements with regard to the transistors and the reproducibility of the transistor characteristic of these transistors is good.

According to Examples 9 and 10, it is found to be possible to provide an organic thin filmtransistor having a good reproducibility and few characteristic variances regardless of different solvents.

Example 11

Two organic thin film transistors are manufactured in the same manner as in Example 7 except that spin coating

is performed using a solution of a mixture solvent of tetrahydrofuran (THF) and paraxylene with a ratio of 8 to 2 containing the polymer 3 in an amount of 1.0 % by weight based on the weight of the solution.

The measuring results of the transistor characteristics of the device are illustrated in Fig. 12.

The electric field-effect mobility of the manufactured organic transistors measured is $1.8 \times 10^{-4}$ $cm^2/Vs$ and $1.3 \times 10^{-4}$ $cm^2/Vs$.

There are few variances among the elements with regard to the transistors and the reproducibility of the transistor characteristic of these transistors is good.

Example 12

Two organic thin film transistors are manufactured in the same manner as in Example 7 except that spin coating is performed using a toluene solution containing the polymer 3 in an amount of 1.0 % by weight based on the weight of the solution.

The electric field-effect mobility of the manufactured organic transistors measured is $1.4 \times 10^{-4}$ $cm^2/Vs$ and $1.7 \times 10^{-4}$ $cm^2/Vs$.

There are few variances among the elements with regard to the transistors and the reproducibility of the transistor characteristic of these transistors is good.

According to Examples 11 and 12, it is found to be possible to provide an organic transistor having a good reproducibility and few characteristic variances regardless of different solvents.

Example 13

Two organic thin film transistors are manufactured in the same manner as in Example 7 except that spin coating is performed using a solution of a mixture solvent of tetrahydrofuran (THF) and paraxylene with a ratio of 8 to 2 containing the polymer 3-2 in an amount of 1.0 % by weight based on the weight of the solution and the channel length between the source electrode and the drain electrode is 50 μm and the channel width is 9 mm.

The measuring results of the transistor characteristics of the device are illustrated in Fig. 13.

The electric field-effect mobility of the manufactured organic thin film transistors measured is $2.0 \times 10^{-4}$ $cm^2/Vs$ and $1.8 \times 10^{-4}$ $cm^2/Vs$.

There are few variances among the elements with regard to the transistors and the reproducibility of the transistor characteristic of these transistors is good.

Example 14

Two organic thin film transistors are manufactured in the same manner as in Example 7 except that spin coating is performed using a toluene solution containing the polymer 3-2 in an amount of 1.0 % by weight based on the weight of the solution and the channel length between the source electrode and the drain electrode is 50 μm and the channel width is 9 mm.

The electric field-effect mobility of the manufactured organic transistors measured is $1.8 \times 10^{-4}$ $cm^2/Vs$ and $1.9 \times 10^{-4}$ $cm^2/Vs$.

There are few variances among the elements with regard to the transistors and the reproducibility of the transistor characteristic of these transistors is good.

According to Examples 15 and 16, it is found to be possible to provide an organic transistor having a good reproducibility and few characteristic variances regardless of different solvents.

Reference Example 3

Two organic thin film transistors are manufactured in the same manner as in Example 7 except that spin coating is performed using a solution of a mixture solvent of tetrahydrofuran (THF) and paraxylene with a ratio of 8 to 2 containing the polymer 5 in an amount of 1.0 % by weight based on the weight of the solution and the channel length between the source electrode and the drain electrode is 50 μm and the channel width is 9 mm.

The measuring results of the transistor characteristics of the device are illustrated in Fig. 14.

The electric field-effect mobility of the manufactured organic transistors measured is $6.1 \times 10^{-4}$ $cm^2/Vs$ and $6.7 \times 10^{-4}$ $cm^2/Vs$.

There are few variances among the elements with regard to the transistors and the reproducibility of the transistor characteristic of these transistors is good.

Reference Example 4

Two organic thin film transistors are manufactured in the same manner as in Example 7 except that spin coating is performed using a toluene solution containing the polymer 5 in an amount of 1.0 % by weight based on the weight of the solution and the channel length between the source electrode and the drain electrode is 50 μm and the channel width is 9 mm.
The electric field-effect mobility of the manufactured organic transistors measured is $6.2 \times 10^{-4}$ $cm^2/Vs$ and $6.4 \times 10^{-4}$ $cm^2/Vs$.
There are few variances among the elements with regard to the transistors and these transistors have transistor characteristics with a good reproducibility.
According to Reference Examples 3 and 4, it is found to be possible to provide an organic transistor having a good reproducibility and few characteristic variances regardless of different solvents.

Example 15

Two organic thin film transistors are manufactured in the same manner as in Example 7 except that spin coating is performed using a solution of a mixture solvent of tetrahydrofuran (THF) and paraxylene with a ratio of 8 to 2 containing the polymer 6 in an amount of 1.0 % by weight based on the weight of the solution.
The measuring results of the transistor characteristics of the device are illustrated in Fig. 15.
The electric field-effect mobility of the manufactured organic transistors measured is $3.9 \times 10^{-5}$ $cm^2/Vs$ and $4.2 \times 10^{-5}$ $cm^2/Vs$.
There are few variances among the elements with regard to the transistors and the reproducibility of the transistor characteristic of these transistors is good.

Example 16

Two organic thin film transistors are manufactured in the same manner as in Example 7 except that spin coating is performed using a toluene solution containing the polymer 6 in an amount of 1.0 % by weight based on the weight of the solution.
The electric field-effect mobility of the manufactured organic transistors measured is $4.0 \times 10^{-5}$ $cm^2/Vs$ and $3.8 \times 10^{-5}$ $cm^2/Vs$.
There are few variances among the elements with regard to the transistors and these transistors have transistor characteristics with a good reproducibility.
According to Examples 15 and 16, it is found to be possible to provide an organic transistor having a good reproducibility and few characteristic variances regardless of different solvents.

Example 17

Two organic thin film transistors are manufactured in the same manner as in Example 7 except that spin coating is performed using a solution of a mixture solvent of tetrahydrofuran (THF) and paraxylene with a ratio of 8 to 2 containing the polymer 7 in an amount of 1.0 % by weight based on the weight of the solution.
The measuring results of the transistor characteristics of the device are illustrated in Fig. 16.
The electric field-effect mobility of the manufactured organic transistors measured is $9.3 \times 10^{-5}$ $cm^2/Vs$ and $8.7 \times 10^{-5}$ $cm^2/Vs$.
There are few variances among the elements with regard to the transistors and the reproducibility of the transistor characteristic of these transistors is good.

Example 18

Two organic thin film transistors are manufactured in the same manner as in Example 7 except that spin coating is performed using a toluene solution containing the polymer 7 in an amount of 1.0 % by weight based on the weight of the solution.
The electric field-effect mobility of the manufactured organic transistors measured is $8.5 \times 10^{-5}$ $cm^2/Vs$ and $8.8 \times 10^{-5}$ $cm^2/Vs$.
There are few variances among the elements with regard to the transistors and these transistors have transistor characteristics with a good reproducibility.
According to Examples 17 and 18, it is found to be possible to provide an organic transistor having a good reproducibility and few characteristic variances regardless of different solvents.

Comparative Example 1

Two organic transistors are manufactured in the same manner as in Example 7 except that a xylene solution containing a copolymer of 9, 9-dioctylfluorene and bithiophene in an amount of 0. 5 % by weight based on the weight of the solution is spin-coated on the $SiO_2$ insulating layer.

The electric field-effect mobility of the manufactured organic thin film transistors measured is $5.1 \times 10^{-4}$ $cm^2/Vs$ and $1.6 \times 10^{-3}$ $cm^2/Vs$.

Comparative Example 2

Two organic transistors are manufactured in the same manner as in Example 7 except that a tetrahydrofuran solution containing a copolymer of 9, 9-dioctylfluorene and bithiophene in an amount of 0. 5 % by weight based on the weight of the solution is spin-coated on the $SiO_2$ insulating layer.

The electric field-effect mobility of the manufactured organic thin film transistors measured is $1.3 \times 10^{-4}$ $cm^2/Vs$ and $5.2 \times 10^{-4}$ $cm^2/Vs$.

As seen in the results, the organic thin film transistors formed using the copolymer of 9,9-dioctylfluorene and bithiophene have a variance in the transistor characteristic. On the other hand, the reproducibility of the transistor characteristic of the organic thin film transistors of the present invention formed using the organic polymer semiconductor containing the arylamine polymer of the present invention is good and is little affected by the selection of the solvent.

That is, it is found that, according to the present invention, an organic thin film transistor having few variances and a good reproducibility for the transistor characteristic can be provided with an easy manufacturing process.

**Claims**

1. An arylamine polymer, comprising:

   a repeating unit represented by the following chemical structure (I),

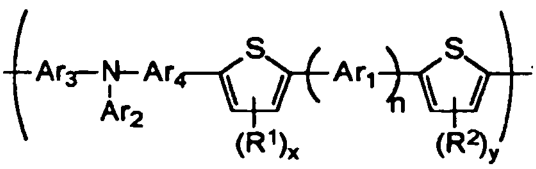

···(I)

   wherein $Ar_1$, $Ar_3$, and $Ar_4$ independently represent a substituted or non-substituted divalent aromatic hydrocarbon group, $Ar_2$ represents a substituted univalent aromatic hydrocarbon group, $R^1$ and $R^2$ independently represent a hydrogen atom, a substituted or non-substituted alkyl group, a substituted or non-substituted alkoxy group or a substituted or non-substituted alkylthio group, x and y independently represent an integer of from 0 to 2, and n represents 1.

2. The arylamine polymer according to Claim 1, wherein the repeating unit represented by the chemical structure (I) is represented by the following chemical structure (II),

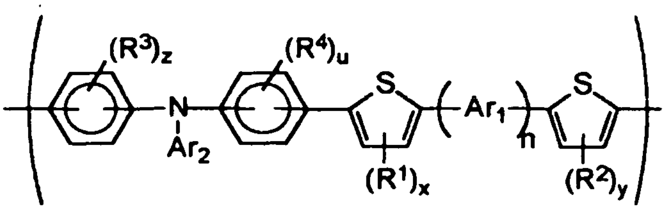

···(II)

wherein $Ar_1$ represents a substituted or non-substituted divalent aromatic hydrocarbon group, $Ar_2$ represents a substituted univalent aromatic hydrocarbon group, $R^1$, $R^2$, $R^3$ and $R^4$ independently represent a hydrogen atom, a substituted or non-substituted alkyl group, a substituted or non-substituted alkoxy group, or a substituted or non-substituted alkylthio group, x and y independently represent an integer of from 0 to 2, z and u independently represent an integer of from 0 to 4, and n represents 1.

**3.** The arylamine polymer according to Claim 1, wherein the repeating unit represented by the chemical structure (I) is represented by the following chemical structure (III),

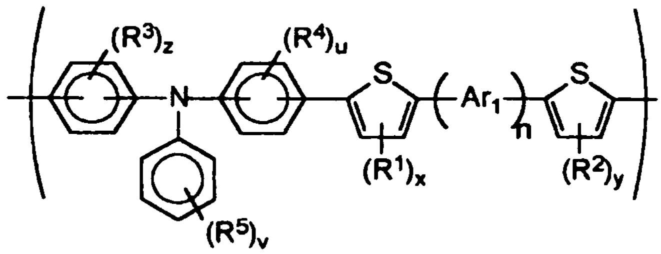

···(III)

wherein $Ar_1$ represents a substituted or non-substituted divalent aromatic hydrocarbon group, $R^1$, $R^2$, $R^3$ and $R^4$, independently represent a hydrogen atom, a substituted or non-substituted alkyl group, a substituted or non-substituted alkoxy group, or a substituted or non-substituted alkylthio group, $R^5$ represents a substituted or non-substituted alkyl group, a substituted or non-substituted alkoxy group, or a substituted or non-substituted alkylthio group, x and y independently represent an integer of from 0 to 2, z and u independently represent an integer of from 0 to 4, v represents an integer of from 1 to 5 and n represents 1.

**4.** An organic thin film transistor comprising:

an organic semiconductor layer comprising the arylamine of Claim 1.

**5.** The organic thin film transistor according to Claim 4, wherein the organic semiconductor layer comprising the arylamine of Claim 2.

**6.** The organic thin film transistor according to Claim 4, wherein the organic semiconductor layer comprising the arylamine of Claim 3.

**7.** The organic thin film transistor according to any one of Claims 4 to 6, further comprising:

a structure comprising a pair of electrodes configured to apply a current through the organic semiconductor layer; and
a third electrode.

**8.** The organic thin film transistor according to Claim 7, wherein the third electrode and the structure are provided with an insulating layer therebetween.

**Patentansprüche**

**1.** Arylamin-Polymer, umfassend:

eine Struktureinheit, die durch die folgende chemische Struktur (I) dargestellt ist,

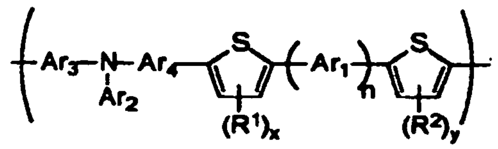

···(I)

worin $Ar_1$, $Ar_3$ und $Ar_4$ unabhängig eine substituierte oder nicht substituierte zweiwertige aromatische Kohlenwasserstoffgruppe sind, $Ar_2$ eine substituierte einwertige aromatische Kohlenwasserstoffgruppe ist, $R^1$ und $R^2$ unabhängig ein Wasserstoffatom, eine substituierte oder nicht substituierte Alkylgruppe, eine substituierte oder nicht substituierte Alkoxygruppe oder eine substituierte oder nicht substituierte Alkylthiogruppe sind, x und y unabhängig eine ganze Zahl von 0 bis 2 sind und n 1 ist.

2. Arylamin-Polymer nach Anspruch 1, wobei die durch die chemische Struktur (I) dargestellte Struktureinheit dargestellt ist durch die folgende chemische Struktur (II),

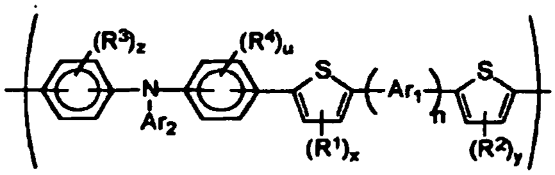

···(II)

worin $Ar_1$ eine substituierte oder nicht substituierte zweiwertige aromatische Kohlenwasserstoffgruppe ist, $Ar_2$ eine substituierte einwertige aromatische Kohlenwasserstoffgruppe ist, $R^1$, $R^2$, $R^3$ und $R^4$ unabhängig ein Wasserstoffatom, eine substituierte oder nicht substituierte Alkylgruppe, eine substituierte oder nicht substituierte Alkoxygruppe oder eine substituierte oder nicht substituierte Alkylthiogruppe sind, x und y unabhängig eine ganze Zahl von 0 bis 2 sind, z und u unabhängig eine ganze Zahl von 0 bis 4 sind und n 1 ist.

3. Arylamin-Polymer nach Anspruch 1, wobei die durch die chemische Struktur (I) dargestellte Struktureinheit dargestellt ist durch die folgende chemische Struktur (III),

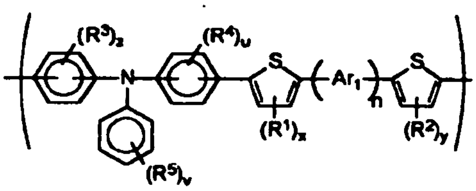

···(III)

wobei $Ar_1$ eine substituierte oder nicht substituierte zweiwertige aromatische Kohlenwasserstoffgruppe ist, $R^1$, $R^2$, $R^3$ und $R^4$ unabhängig ein Wasserstoffatom, eine substituierte oder nicht substituierte Alkylgruppe, eine substituierte oder nicht substituierte Alkoxygruppe oder eine substituierte oder nicht substituierte Alkylthiogruppe sind, $R^5$ eine substituierte oder nicht substituierte Alkylgruppe, eine substituierte oder nicht substituierte Alkoxygruppe oder eine substituierte oder nicht substituierte Alkylthiogruppe ist, x und y unabhängig eine ganze Zahl von 0 bis 2 sind, z und u unabhängig eine ganze Zahl von 0 bis 4 sind, v eine ganze Zahl von 1 bis 5 ist und n 1 ist.

4. Organischer Dünnschichttransistor, umfassend eine organische Halbleiterschicht, die das Arylamin nach Anspruch 1 umfasst.

5. Organischer Dünnschichttransistor nach Anspruch 4, wobei die organische Halbleiterschicht das Arylamin nach Anspruch 2 umfasst.

6. Organischer Dünnschichttransistor nach Anspruch 4, wobei die organische Halbleiterschicht das Arylamin nach Anspruch 3 umfasst.

**7.** Organischer Dünnschichttransistor nach irgendeinem der Ansprüche 4 bis 6, ferner umfassend:

eine Struktur umfassend ein Paar von Elektroden, die konfiguriert sind, um einen Strom durch die organische Halbleiterschicht anzulegen; und
eine dritte Elektrode.

**8.** Organischer Dünnschichttransistor nach Anspruch 7, wobei die dritte Elektrode und die Struktur mit einer Isolierschicht dazwischen vorgesehen sind.

**Revendications**

**1.** Polymère d'arylamine, comprenant :

une unité répétitive représentée par la structure chimique (I) suivante,

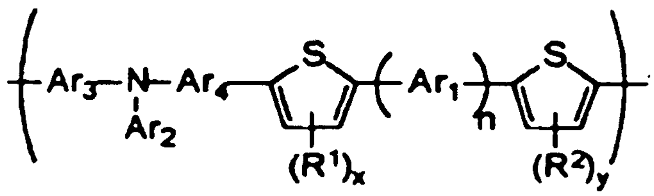

···(I)

où $Ar_1$, $Ar_3$ et $Ar_4$ représentent indépendamment un groupe hydrocarboné aromatique divalent substitué ou non substitué, $Ar_2$ représente un groupe hydrocarboné aromatique univalent substitué, $R^1$ et $R^2$ représentent indépendamment un atome d'hydrogène, un groupe alkyle substitué ou non substitué, un groupe alcoxy substitué ou non substitué ou un groupe alkylthio substitué ou non substitué, x et y représentent indépendamment un nombre entier de 0 à 2, et n représente 1.

**2.** Polymère d'arylamine selon la revendication 1, dans lequel l'unité répétitive représentée par la structure chimique (I) est représentée par la structure chimique (II) suivante,

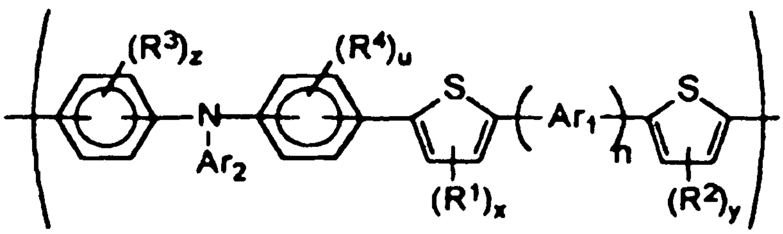

···(II)

où $Ar_1$ représente un groupe hydrocarboné aromatique divalent substitué ou non substitué, $Ar_2$ représente un groupe hydrocarboné aromatique univalent substitué, $R^1$, $R^2$, $R^3$ et $R^4$ représentent indépendamment un atome d'hydrogène, un groupe alkyle substitué ou non substitué, un groupe alcoxy substitué ou non substitué, ou un groupe alkylthio substitué ou non substitué, x et y représentent indépendamment un nombre entier de 0 à 2, z et u représentent indépendamment un nombre entier de 0 à 4, et n représente 1.

**3.** Polymère d'arylamine selon la revendication 1, dans lequel l'unité répétitive représentée par la structure chimique (I) est représentée par la structure chimique (III) suivante,

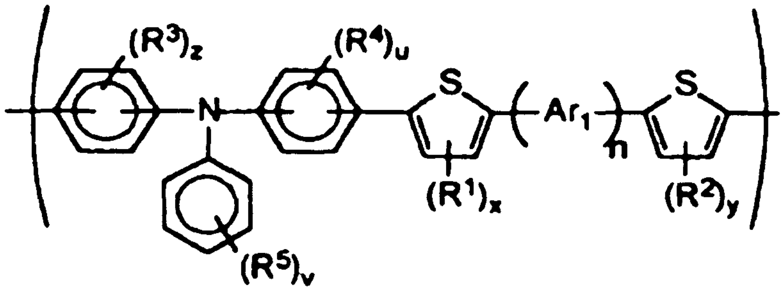

...(III)

où $Ar_1$ représente un groupe hydrocarboné aromatique divalent substitué ou non substitué, $R^1$, $R^2$, $R^3$ et $R^4$ représentent indépendamment un atome d'hydrogène, un groupe alkyle substitué ou non substitué, un groupe alcoxy substitué ou non substitué, ou un groupe alkylthio substitué ou non substitué, $R^5$ représente un groupe alkyle substitué ou non substitué, un groupe alcoxy substitué ou non substitué, ou un groupe alkylthio substitué ou non substitué, x et y représentent indépendamment un nombre entier de 0 à 2, z et u représentent indépendamment un nombre entier de 0 à 4, v représente un nombre entier de 1 à 5 et n représente 1.

**4.** Transistor à mince film organique comprenant :

une couche de semi-conducteur organique comprenant l'arylamine selon la revendication 1.

**5.** Transistor à mince film organique selon la revendication 4, dans lequel la couche de semi-conducteur organique comprend l'arylamine selon la revendication 2.

**6.** Transistor à mince film organique selon la revendication 4, dans lequel la couche de semi-conducteur organique comprend l'arylamine selon la revendication 3.

**7.** Transistor à mince film organique selon l'une quelconque des revendications 4 à 6, comprenant de plus :

une structure comprenant une paire d'électrodes configurées pour appliquer un courant à travers la couche de semi-conducteur organique ; et
une troisième électrode.

**8.** Transistor à mince film organique selon la revendication 7, dans lequel la troisième électrode et la structure sont munies d'une couche isolante entre celles-ci.

FIG. 1A

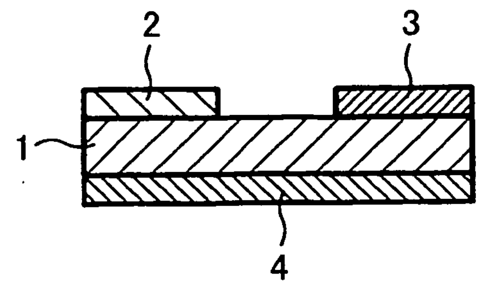


FIG. 1B

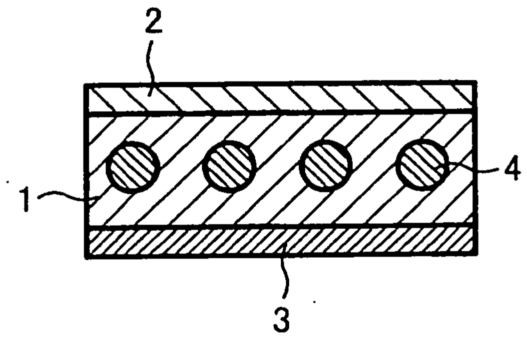


FIG. 1C

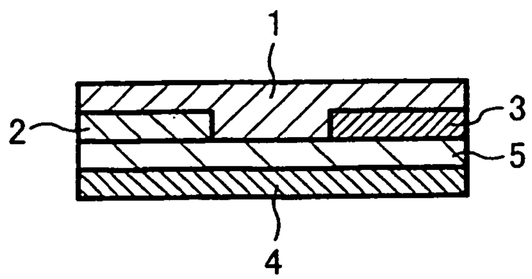


FIG. 1D

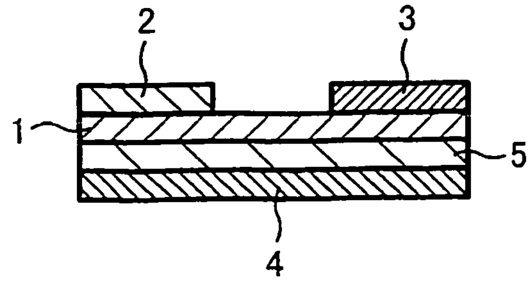

FIG. 2

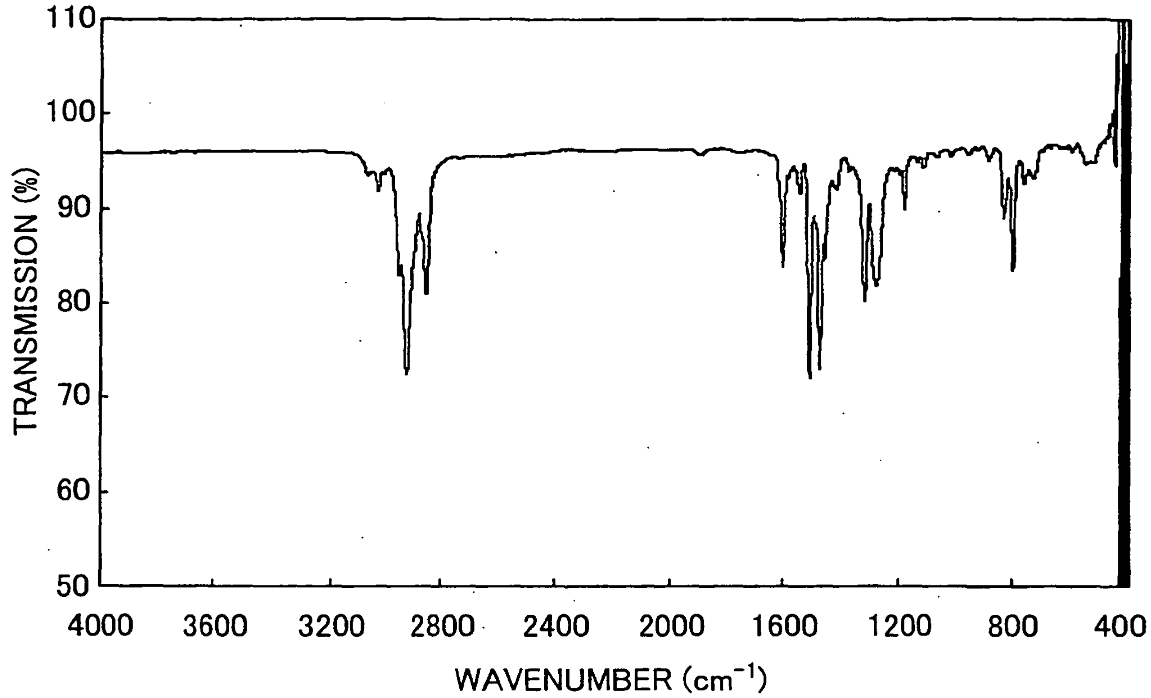
110
100
90
80
70
60
50
TRANSMISSION (%)
4000
3600
3200
2800
2400
2000
1600
1200
800
400
WAVENUMBER ($cm^{-1}$)

FIG. 3

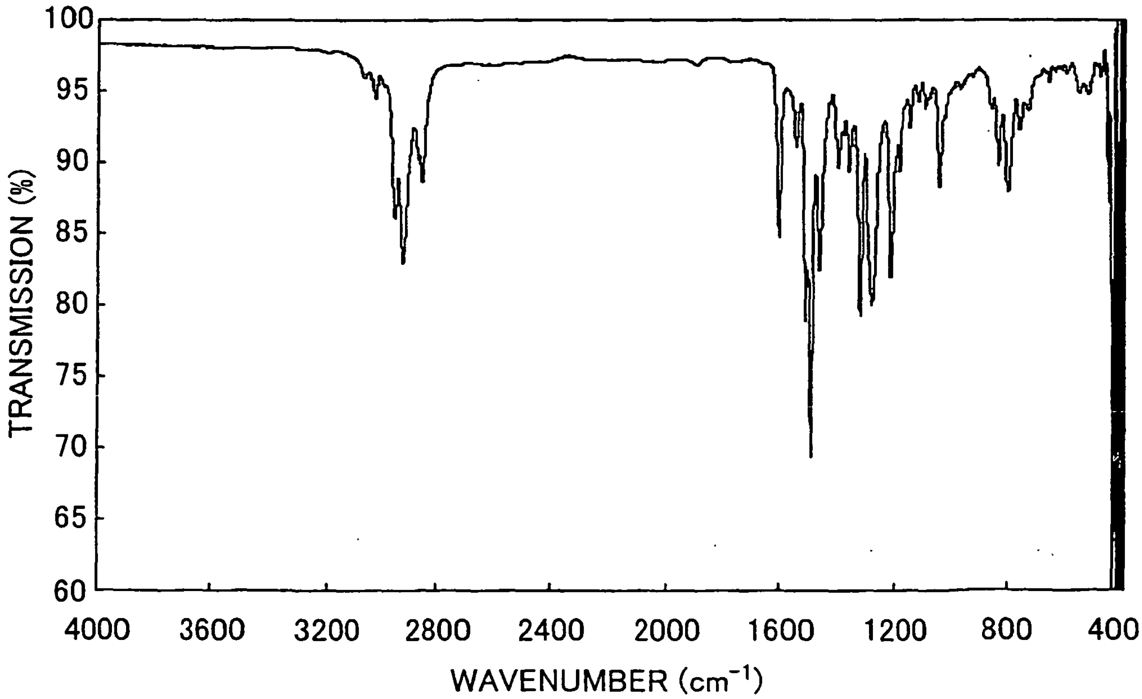
100
95
90
85
80
75
70
65
60
TRANSMISSION (%)
4000
3600
3200
2800
2400
2000
1600
1200
800
400
WAVENUMBER ($cm^{-1}$)

FIG. 4

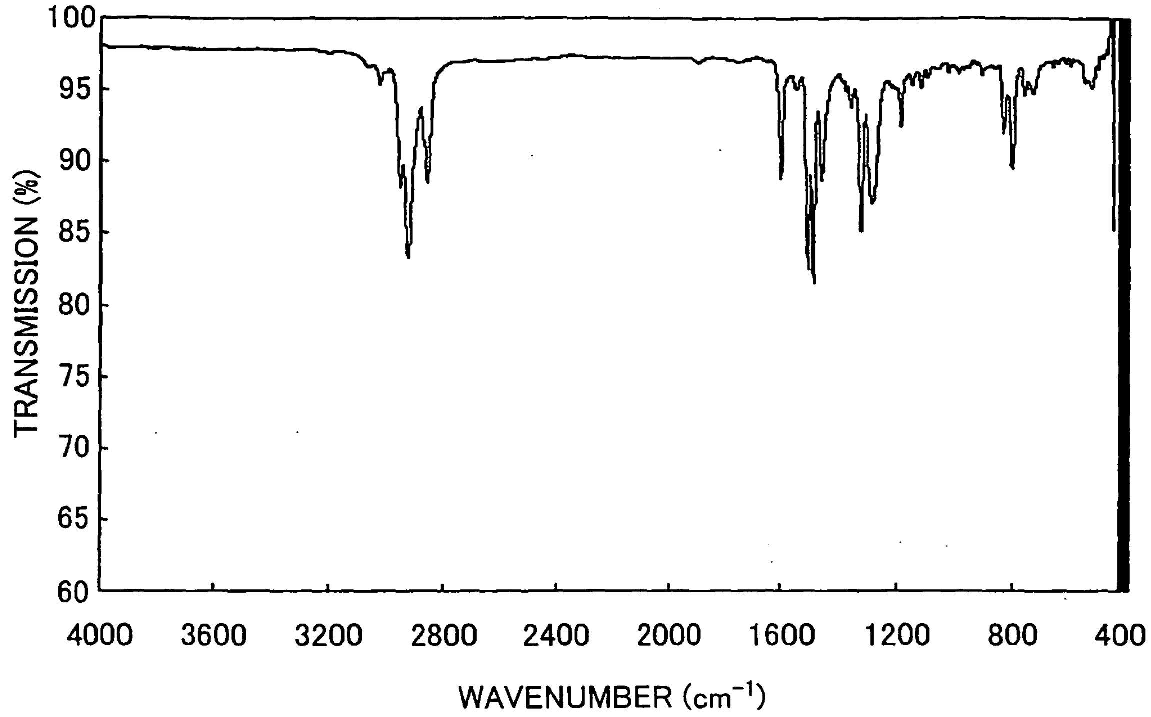
TRANSMISSION (%)
100
95
90
85
80
75
70
65
60
4000
3600
3200
2800
2400
2000
1600
1200
800
400
WAVENUMBER ($cm^{-1}$)

FIG. 5

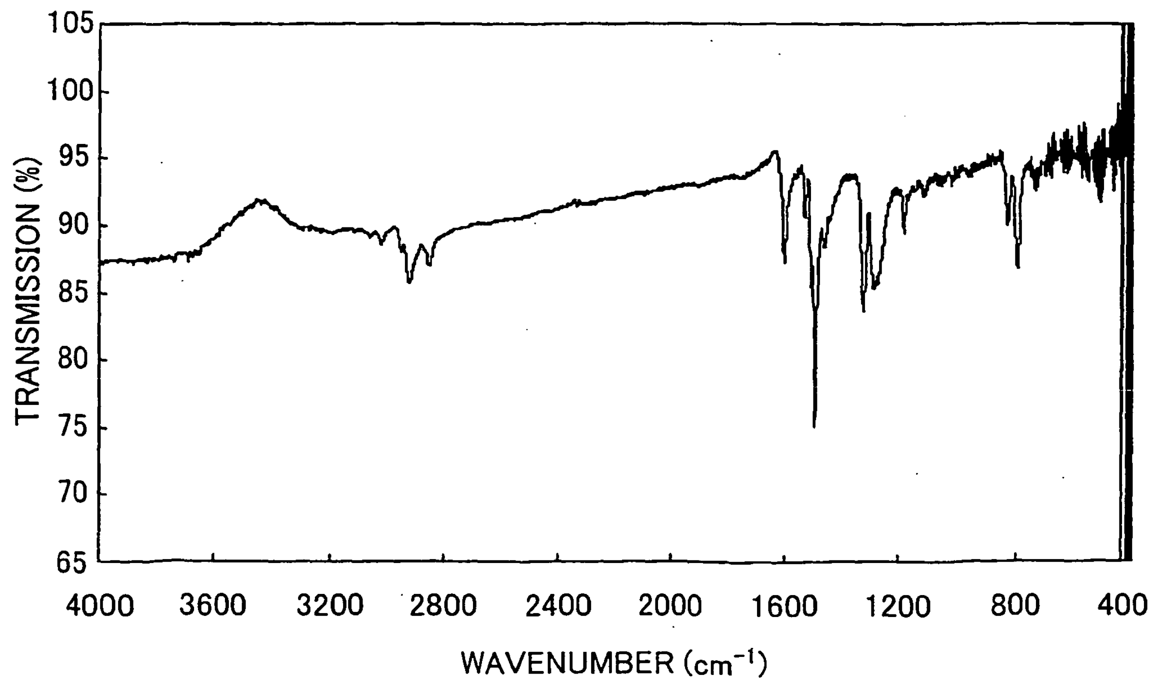
TRANSMISSION (%)
105
100
95
90
85
80
75
70
65
4000
3600
3200
2800
2400
2000
1600
1200
800
400
WAVENUMBER ($cm^{-1}$)

FIG. 6

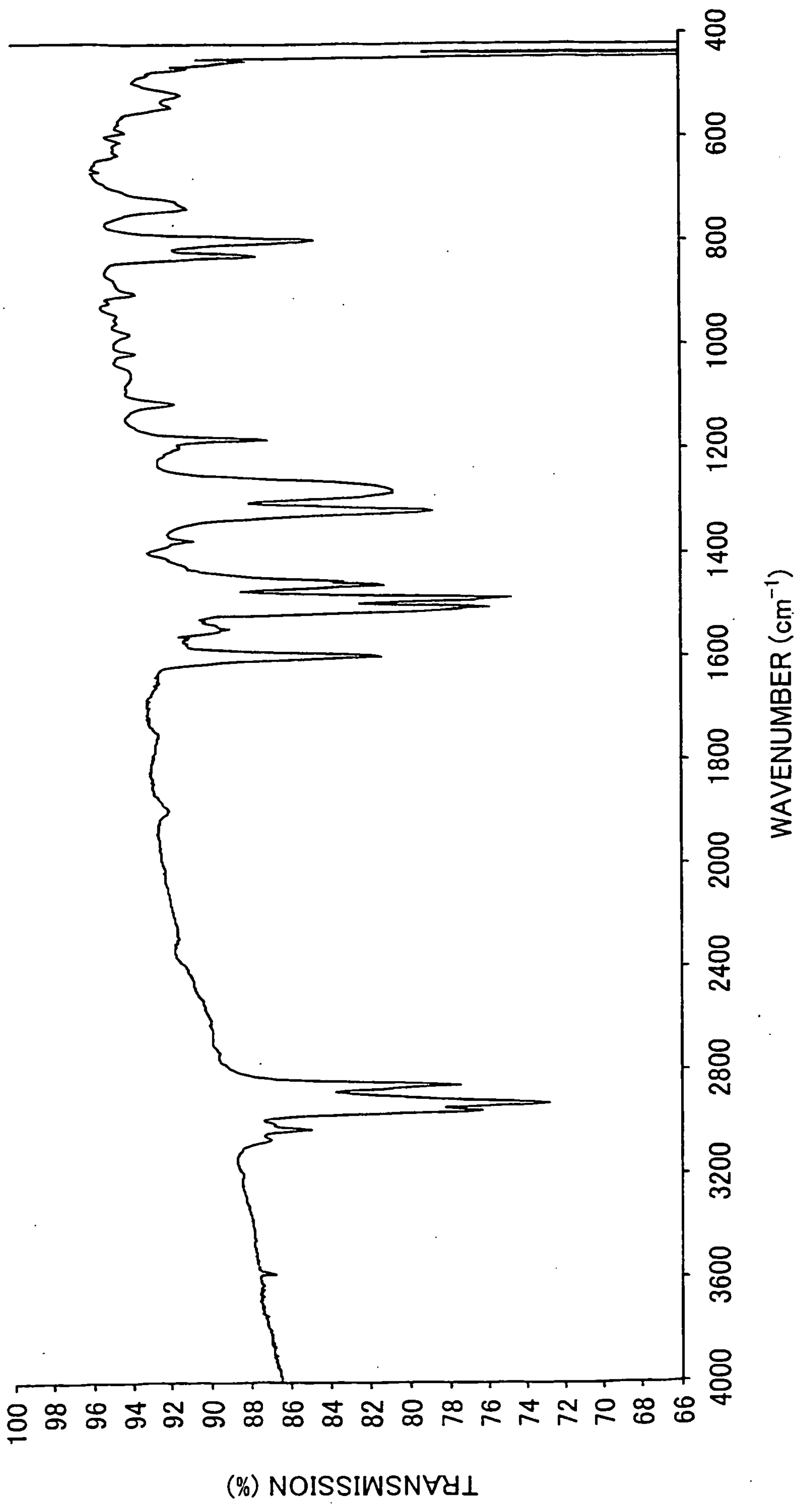
TRANSMISSION (%)
100 98 96 94 92 90 88 86 84 82 80 78 76 74 72 70 68 66
WAVENUMBER (cm$^{-1}$)
4000 3600 3200 2800 2400 2000 1800 1600 1400 1200 1000 800 600 400

FIG. 7

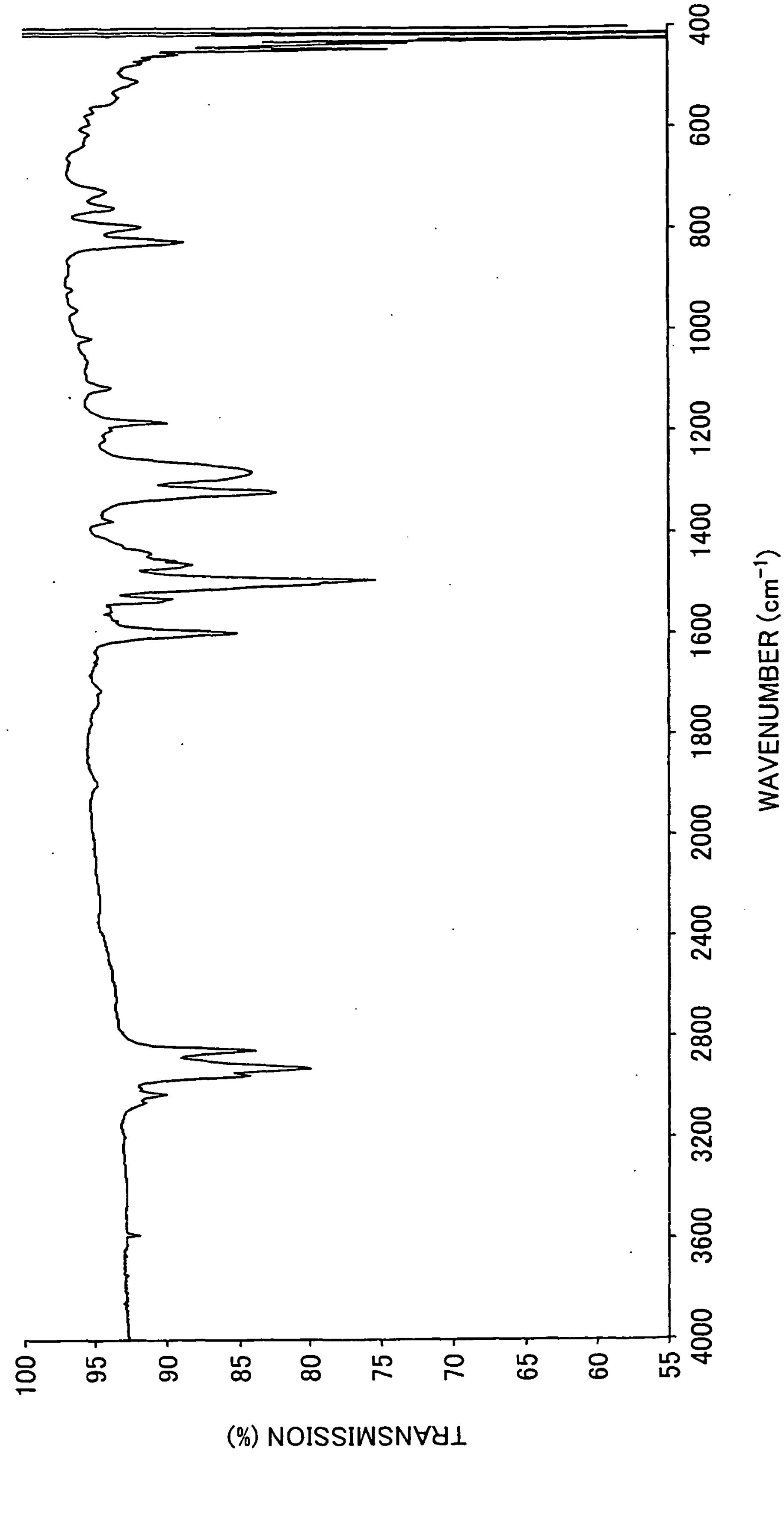
TRANSMISSION (%)
100
95
90
85
80
75
70
65
60
55
WAVENUMBER (cm$^{-1}$)
4000
3600
3200
2800
2400
2000
1800
1600
1400
1200
1000
800
600
400

FIG. 8

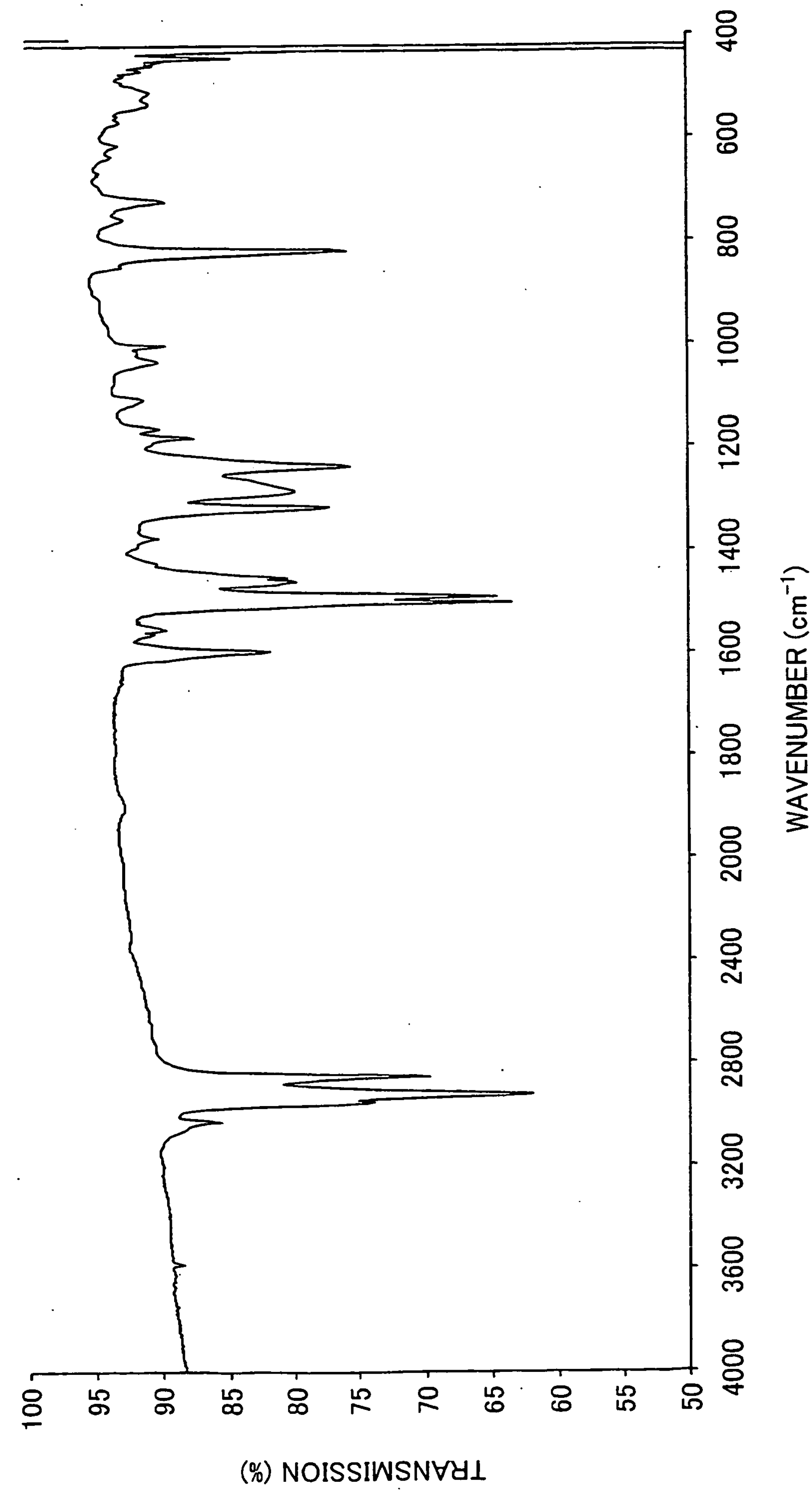
TRANSMISSION (%)
100
95
90
85
80
75
70
65
60
55
50
WAVENUMBER (cm$^{-1}$)
4000
3600
3200
2800
2400
2000
1800
1600
1400
1200
1000
800
600
400

FIG. 9

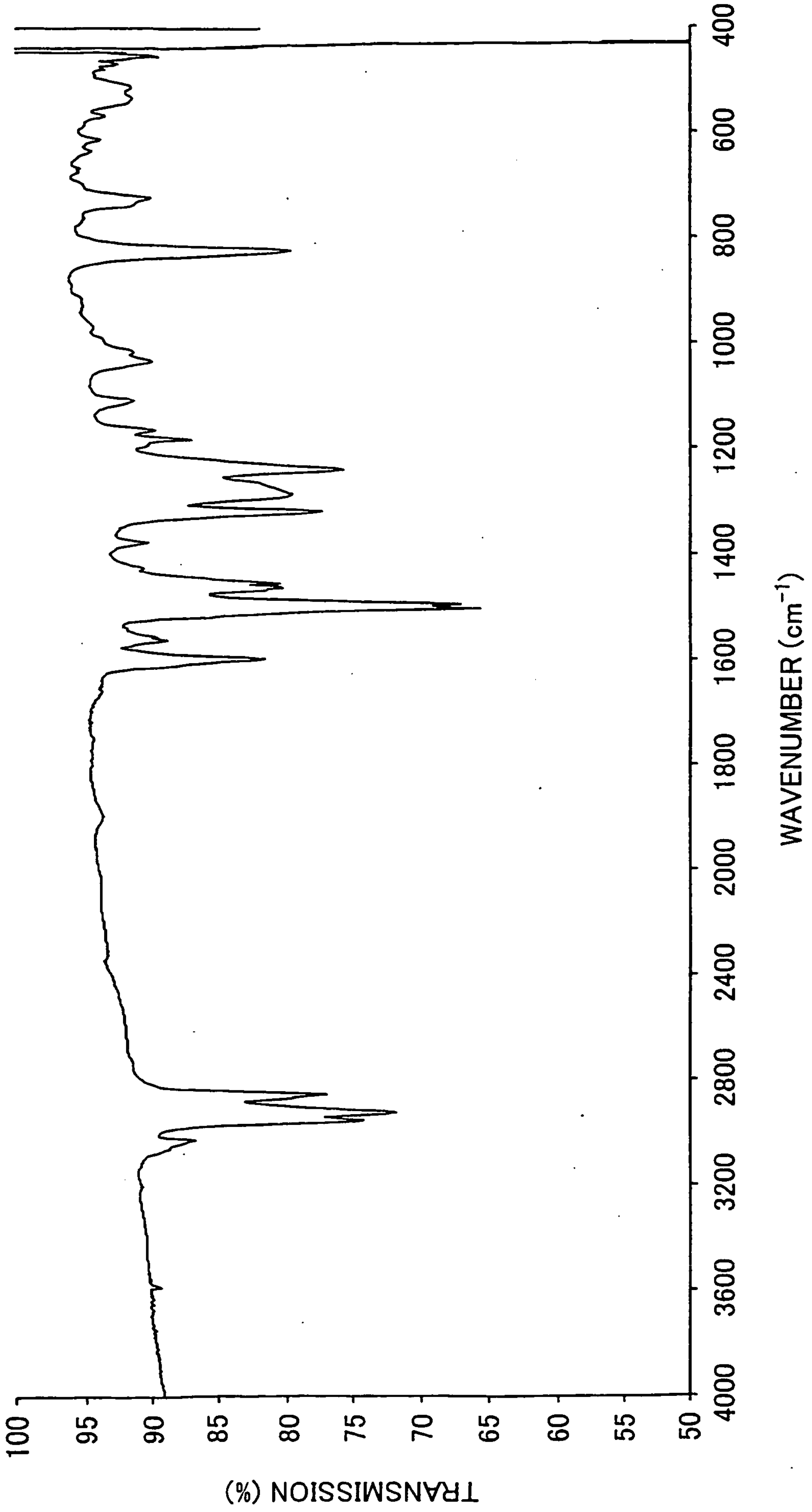
TRANSMISSION (%)
100
95
90
85
80
75
70
65
60
55
50
WAVENUMBER ($cm^{-1}$)
4000
3600
3200
2800
2400
2000
1800
1600
1400
1200
1000
800
600
400

FIG. 10

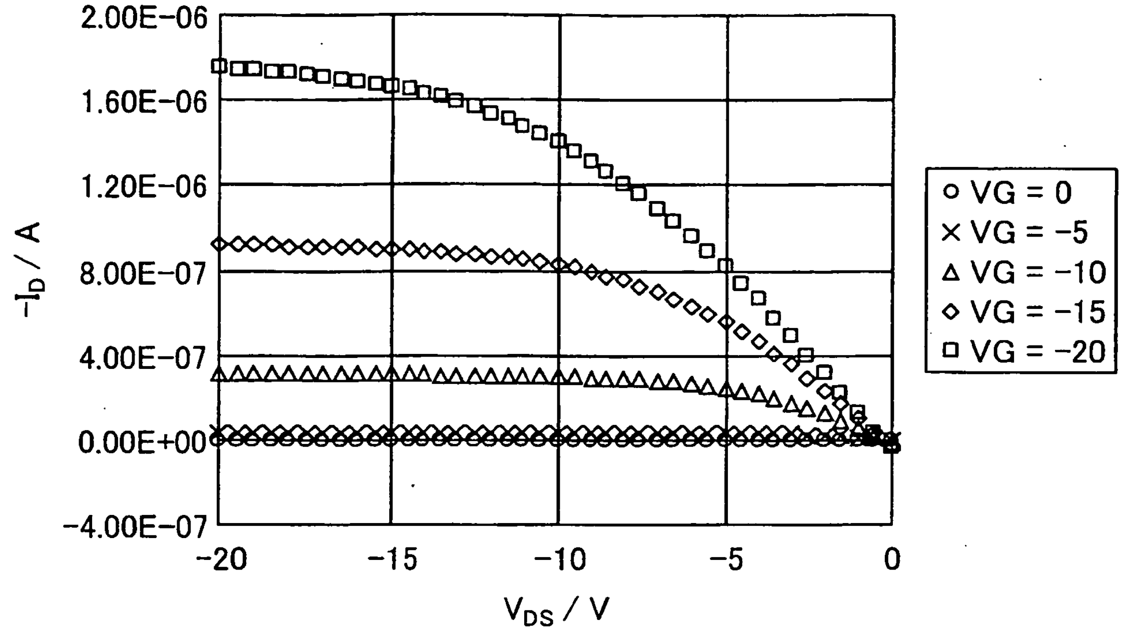
2.00E-06
1.60E-06
1.20E-06
8.00E-07
4.00E-07
0.00E+00
-4.00E-07
$-I_D$ / A
-20
-15
-10
-5
0
$V_{DS}$ / V
VG = 0
VG = -5
VG = -10
VG = -15
VG = -20

FIG. 11

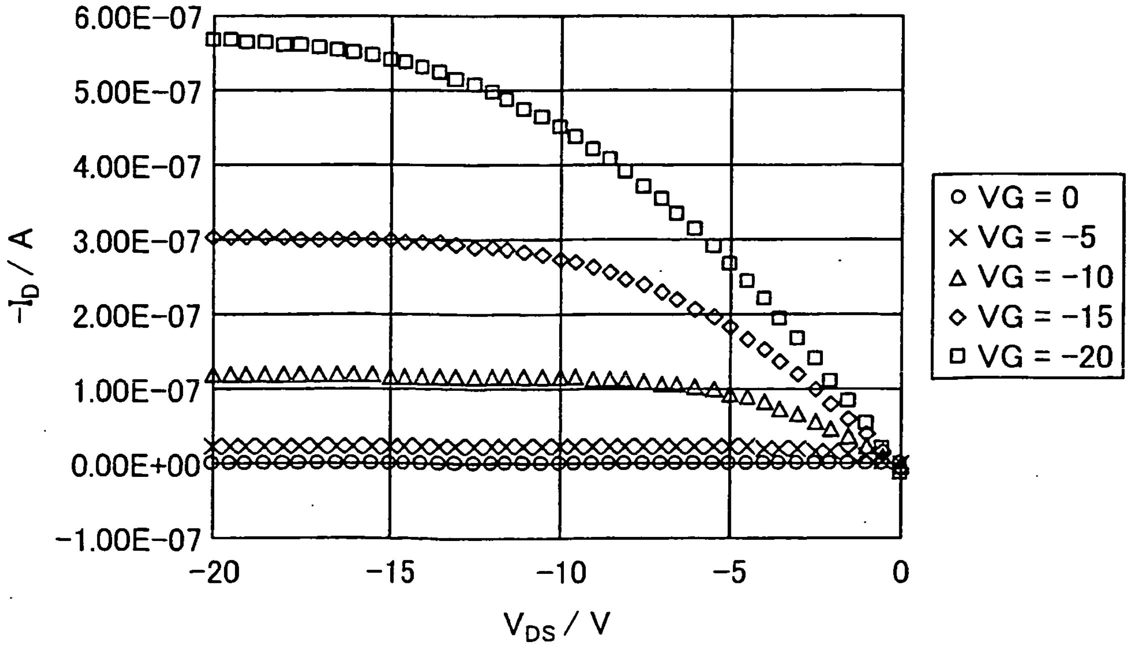
6.00E-07
5.00E-07
4.00E-07
3.00E-07
2.00E-07
1.00E-07
0.00E+00
-1.00E-07
$-I_D$ / A
-20
-15
-10
-5
0
$V_{DS}$ / V
VG = 0
VG = -5
VG = -10
VG = -15
VG = -20

# FIG. 12

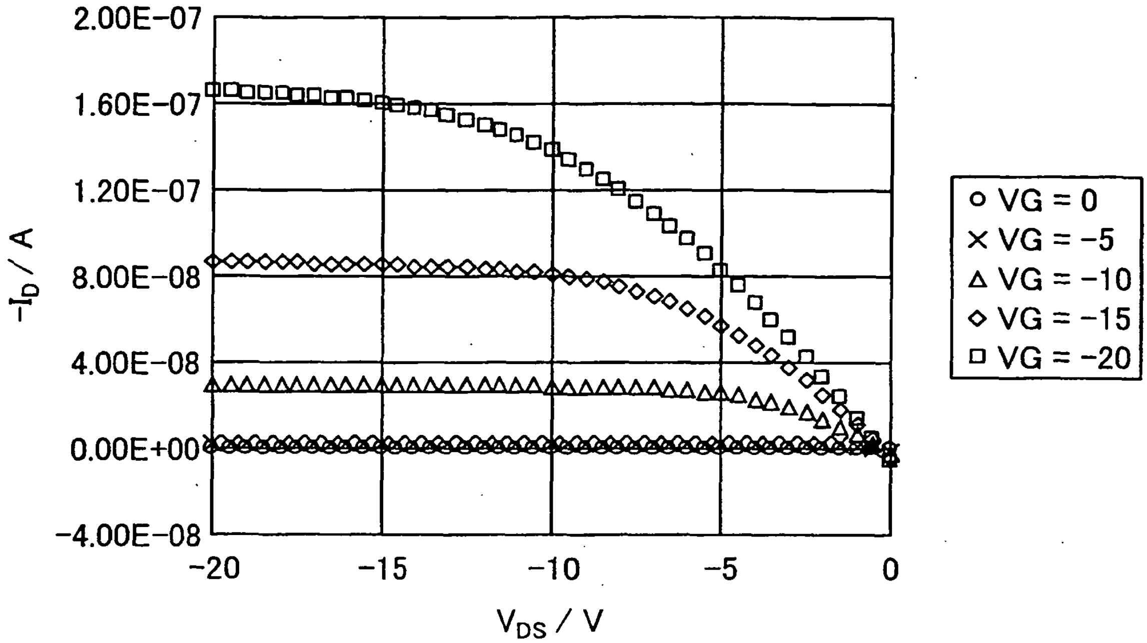
2.00E-07
1.60E-07
1.20E-07
8.00E-08
4.00E-08
0.00E+00
-4.00E-08
$-I_D$ / A
-20
-15
-10
-5
0
$V_{DS}$ / V
○ VG = 0
× VG = -5
△ VG = -10
◇ VG = -15
□ VG = -20

# FIG. 13

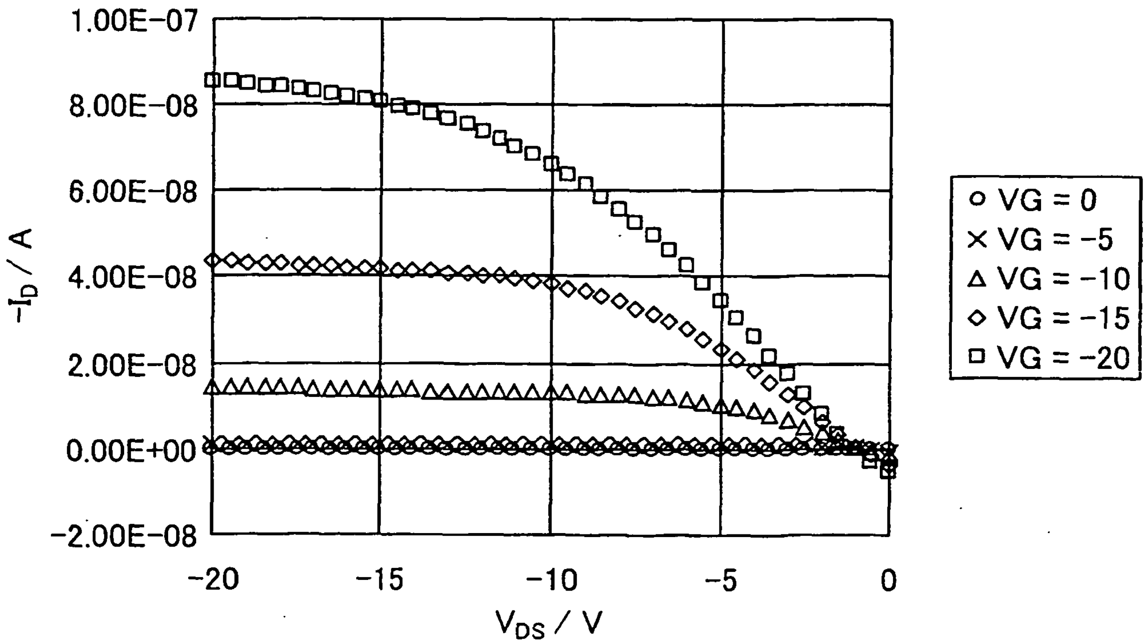
1.00E-07
8.00E-08
6.00E-08
4.00E-08
2.00E-08
0.00E+00
-2.00E-08
$-I_D$ / A
-20
-15
-10
-5
0
$V_{DS}$ / V
○ VG = 0
× VG = -5
△ VG = -10
◇ VG = -15
□ VG = -20

FIG. 14

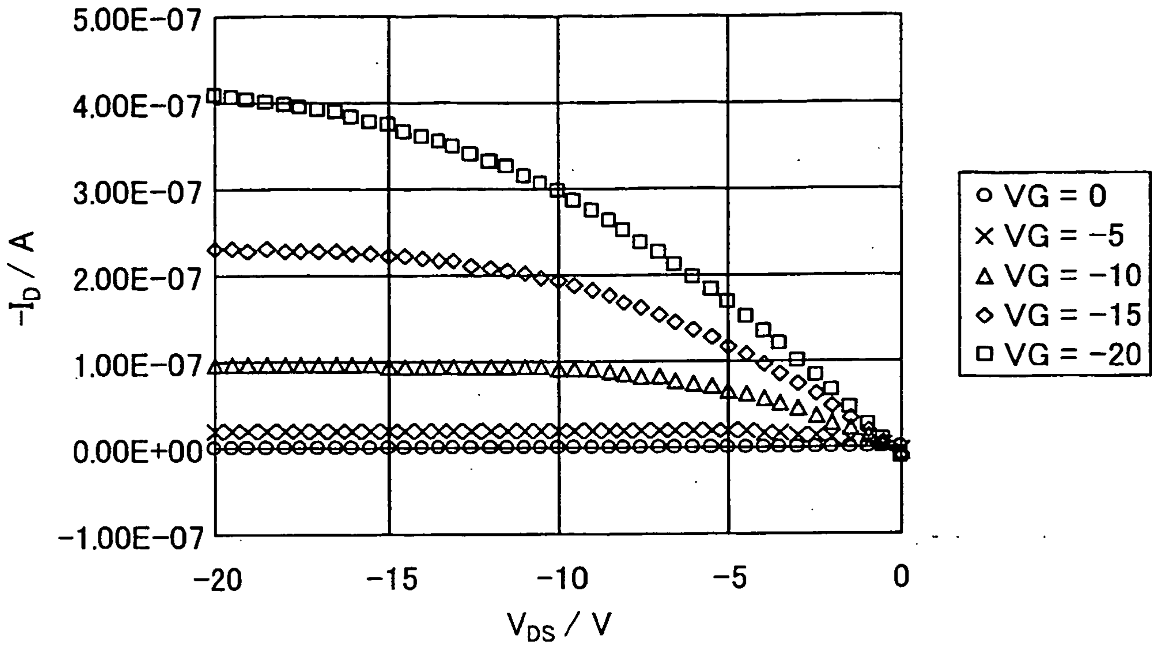
5.00E-07
4.00E-07
3.00E-07
2.00E-07
1.00E-07
0.00E+00
-1.00E-07
$-I_D$ / A
-20
-15
-10
-5
0
$V_{DS}$ / V
○ VG = 0
× VG = -5
△ VG = -10
◇ VG = -15
□ VG = -20

FIG. 15

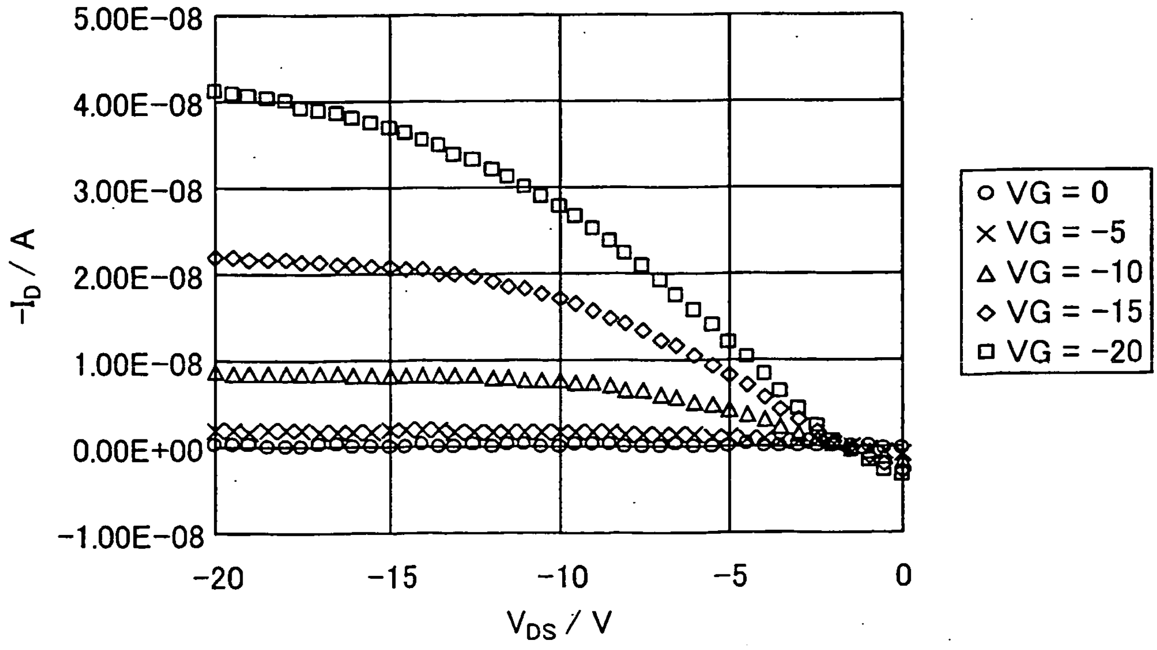
5.00E-08
4.00E-08
3.00E-08
2.00E-08
1.00E-08
0.00E+00
-1.00E-08
$-I_D$ / A
-20
-15
-10
-5
0
$V_{DS}$ / V
○ VG = 0
× VG = -5
△ VG = -10
◇ VG = -15
□ VG = -20

FIG. 16

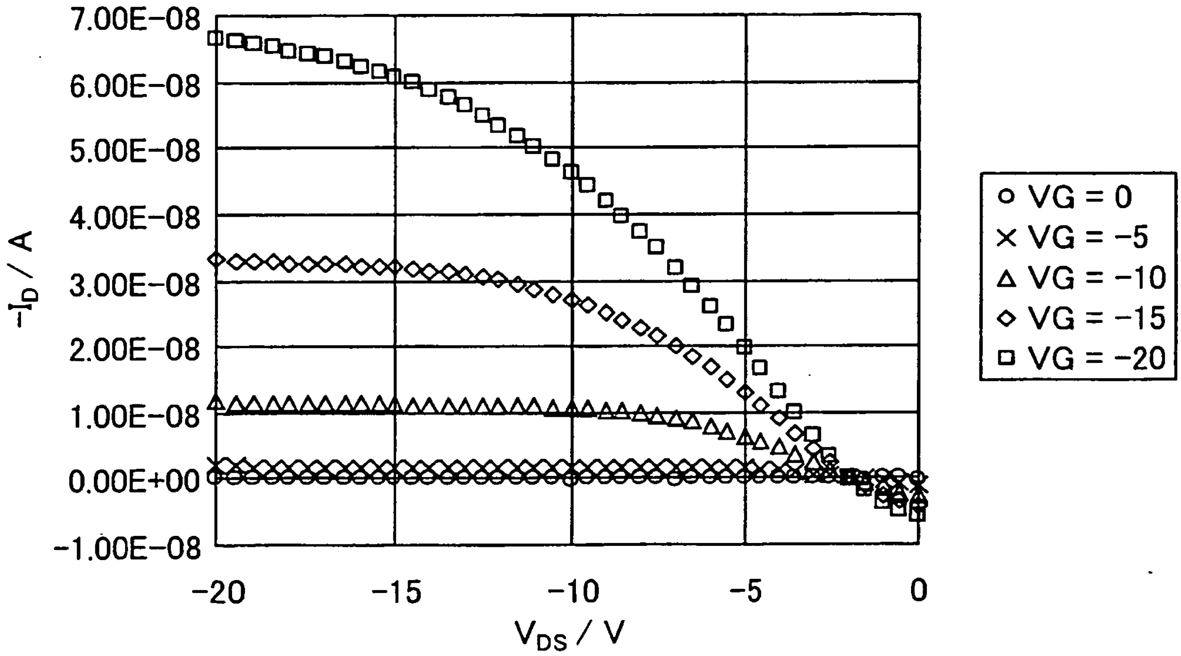
7.00E−08
6.00E−08
5.00E−08
4.00E−08
3.00E−08
2.00E−08
1.00E−08
0.00E+00
−1.00E−08
$-I_D$ / A
−20
−15
−10
−5
0
$V_{DS}$ / V
VG = 0
VG = −5
VG = −10
VG = −15
VG = −20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9920675 A
- WO 9709394 A
- WO 03035714 A
- JP H10310635 B
- JP H08157575 B
- WO 0079617 A


### Non-patent literature cited in the description


- *Synth. Met.,* 1997, vol. 84, 269
- *Appl. Phys. Lett.,* 1996, vol. 69 (26), 4108
- *Science,* 2000, vol. 290, 2123
- **YAMAMOTO, K. et al.** Synthesis and Electrochemical Properties of Oligo- and Poly(thienylphenylamine)s. *Macromolecules,* vol. 35 (15), 5782-5788